# EUROPEAN PATENT APPLICATION

(11) **EP 1 837 345 A1**
(43) Date of publication of application: **26.09.2007**
(21) Application number: 06006041.5
(22) Date of filing: 23.03.2006
(51) Int. Cl.: C07K 14/205, C12N 9/10, A61K 38/00

(54) **Pharmaceutical compositions comprising cholesteryl-alpha-glucosyltransferase for the treatment of Helicobacteri pylori infections**

(71) Applicant: Max-Planck-Gesellschaft zur Förderung der Wissenschaften e.V., 80539 München (DE); Universität Hamburg, 20146 Hamburg (DE)
(72) Inventor: Wunder, Christian, 07743 Jena (DE); Churin, Yuri, 13055 Berlin (DE); Meyer, Thomas F., 14612 Falkensee (DE); Warnecke, Dirk, Dr., 22607 Hamburg (DE); Lebrun, Anne-Hélène, 20537 Hamburg (DE); Hildebrand, Janosch, 22083 Hamburg (DE)
(74) Representative: Weiss, Wolfgang

(57) **Abstract**

The present invention refers to pharmaceutical compositions comprising cholesteryl-α-glucosyltransferase from Helicobacter, inhibitors of cholesteryl-α-glucosyltransferase and the use thereof for the manufacture of a medicament for the prevention or/and treatment of *Helicobacter* infection or/and a *Helicobacter*-associated disease. Another subject is the screening method for novel inhibitors of cholesteryl-α-glucosyltransferase.

SEQUENCE LISTING

<110> Max-Planck-Gesellschaft zur Foerderung der Wissenschaften
e.V.

<120> Inhibition of cholesteryl-alpha-glucosyl-transferase for the
treatment of H. pylori infections

<130> 37479PEP

<160> 12

<170> PatentIn version 3.3

<210> 1
<211> 1170
<212> DNA
<213> Helicobacter pylori strain 26695

<220>
<221> CDS
<222> (1)..(1170)

<400> 1
atg gtt att gtt tta gtc gtg gat agt ttt aaa gac acc agt aat ggc 48
Met Val Ile Val Leu Val Val Asp Ser Phe Lys Asp Thr Ser Asn Gly
1               5                   10                  15

act tct atg aca gcg ttt cgt ttt ttt gaa gcg ctg aaa aaa aga ggg 96
Thr Ser Met Thr Ala Phe Arg Phe Phe Glu Ala Leu Lys Lys Arg Gly
            20                  25                  30

cat gtg atg aga gtg gtc gcc cct cat gtg gat aat tta ggg agt gaa 144
His Val Met Arg Val Val Ala Pro His Val Asp Asn Leu Gly Ser Glu
        35                  40                  45

gaa gag ggg tat tac aac ctt aaa gag cgc tac atc ccc cta gtt aca 192
Glu Glu Gly Tyr Tyr Asn Leu Lys Glu Arg Tyr Ile Pro Leu Val Thr
    50                  55                  60

gaa att tca cac aaa caa cac atc ctt ttt gct aaa ccc gat gaa aaa 240
Glu Ile Ser His Lys Gln His Ile Leu Phe Ala Lys Pro Asp Glu Lys
65                  70                  75                  80

atc tta aga aag gct ttt aag gga gcg gat atg atc cat act tat ttg 288
Ile Leu Arg Lys Ala Phe Lys Gly Ala Asp Met Ile His Thr Tyr Leu
                85                  90                  95

cct ttt ttg cta gaa aaa aca gcc gta aaa atc gcg cga gaa atg caa 336
Pro Phe Leu Leu Glu Lys Thr Ala Val Lys Ile Ala Arg Glu Met Gln
            100                 105                 110

gtg cct tat att ggc tct ttc cat tta cag cca gag cat att tct tat 384
Val Pro Tyr Ile Gly Ser Phe His Leu Gln Pro Glu His Ile Ser Tyr
        115                 120                 125

aac atg aaa ttg ggg tgg ttt tct tgg ttc aac atg atg ctt ttt tcg 432
Asn Met Lys Leu Gly Trp Phe Ser Trp Phe Asn Met Met Leu Phe Ser
    130                 135                 140

tgg ttt aaa tct tcg cat tac cgc tat atc cac cat atc cat tgc ccg 480
Trp Phe Lys Ser Ser His Tyr Arg Tyr Ile His His Ile His Cys Pro
145                 150                 155                 160

tca aaa ttc att gta gaa gaa tta gaa aaa tac aac tat gga ggg aaa 528
Ser Lys Phe Ile Val Glu Glu Leu Glu Lys Tyr Asn Tyr Gly Gly Lys
                165                 170                 175

aaa tac gct att tct aac ggc ttt gat ccc atg ttt aga ttt gaa cac     576
Lys Tyr Ala Ile Ser Asn Gly Phe Asp Pro Met Phe Arg Phe Glu His
        180                 185                 190

ccg caa aaa agc ctt ttt gac acc aca ccc ttt aaa atc gct atg gta     624
Pro Gln Lys Ser Leu Phe Asp Thr Thr Pro Phe Lys Ile Ala Met Val
        195                 200                 205

gga cgc tat tct aat gaa aaa aat caa agc gtt tta atc aaa gcg gtt     672
Gly Arg Tyr Ser Asn Glu Lys Asn Gln Ser Val Leu Ile Lys Ala Val
    210                 215                 220

gct tta agc aaa tac aaa caa gat att gta tta ttg ctc aaa ggc aaa     720
Ala Leu Ser Lys Tyr Lys Gln Asp Ile Val Leu Leu Leu Lys Gly Lys
225                 230                 235                 240

ggg cct gat gag aaa aaa atc aaa ctt tta gcc caa aaa cta ggc gta     768
Gly Pro Asp Glu Lys Lys Ile Lys Leu Leu Ala Gln Lys Leu Gly Val
                245                 250                 255

aaa gcg gag ttt ggg ttt gtc aat tcc aat gaa ttg tta gag atc tta     816
Lys Ala Glu Phe Gly Phe Val Asn Ser Asn Glu Leu Leu Glu Ile Leu
            260                 265                 270

aaa act tgc acc ctt tat gtg cat gca gcc aat gtg gaa agc gaa gcg     864
Lys Thr Cys Thr Leu Tyr Val His Ala Ala Asn Val Glu Ser Glu Ala
        275                 280                 285

att gcg tgc tta gag gcc att agc gtg ggg att gtg cct gtt atc gct     912
Ile Ala Cys Leu Glu Ala Ile Ser Val Gly Ile Val Pro Val Ile Ala
    290                 295                 300

aat agc cct tta agc gcg acc agg caa ttt gcg cta gat gaa cga tcg     960
Asn Ser Pro Leu Ser Ala Thr Arg Gln Phe Ala Leu Asp Glu Arg Ser
305                 310                 315                 320

cta ttt gaa cct aat aac gct aaa gat ttg agc gct aaa ata gat tgg     1008
Leu Phe Glu Pro Asn Asn Ala Lys Asp Leu Ser Ala Lys Ile Asp Trp
                325                 330                 335

tgg tta gaa aac aag ctt gaa aga gaa agg atg caa aac gaa tac gct     1056
Trp Leu Glu Asn Lys Leu Glu Arg Glu Arg Met Gln Asn Glu Tyr Ala
            340                 345                 350

aaa agc gct tta aat tac act tta gaa aat tca gtc att caa att gaa     1104
Lys Ser Ala Leu Asn Tyr Thr Leu Glu Asn Ser Val Ile Gln Ile Glu
        355                 360                 365

aaa gtt tat gaa gaa gcg atc aga gat ttt aaa aat aac ccc cat ctc     1152
Lys Val Tyr Glu Glu Ala Ile Arg Asp Phe Lys Asn Asn Pro His Leu
    370                 375                 380

ttt aaa acc tta tca taa                                             1170
Phe Lys Thr Leu Ser
385

<210>  2
<211>  389
<212>  PRT
<213>  Helicobacter pylori strain 26695

<400>  2

Met Val Ile Val Leu Val Val Asp Ser Phe Lys Asp Thr Ser Asn Gly
1               5                   10                  15

Thr Ser Met Thr Ala Phe Arg Phe Phe Glu Ala Leu Lys Lys Arg Gly

20                  25                  30

His Val Met Arg Val Val Ala Pro His Val Asp Asn Leu Gly Ser Glu
        35                  40                  45

Glu Glu Gly Tyr Tyr Asn Leu Lys Glu Arg Tyr Ile Pro Leu Val Thr
    50                  55                  60

Glu Ile Ser His Lys Gln His Ile Leu Phe Ala Lys Pro Asp Glu Lys
65                  70                  75                  80

Ile Leu Arg Lys Ala Phe Lys Gly Ala Asp Met Ile His Thr Tyr Leu
                85                  90                  95

Pro Phe Leu Leu Glu Lys Thr Ala Val Lys Ile Ala Arg Glu Met Gln
            100                 105                 110

Val Pro Tyr Ile Gly Ser Phe His Leu Gln Pro Glu His Ile Ser Tyr
        115                 120                 125

Asn Met Lys Leu Gly Trp Phe Ser Trp Phe Asn Met Met Leu Phe Ser
    130                 135                 140

Trp Phe Lys Ser Ser His Tyr Arg Tyr Ile His His Ile His Cys Pro
145                 150                 155                 160

Ser Lys Phe Ile Val Glu Glu Leu Glu Lys Tyr Asn Tyr Gly Gly Lys
                165                 170                 175

Lys Tyr Ala Ile Ser Asn Gly Phe Asp Pro Met Phe Arg Phe Glu His
            180                 185                 190

Pro Gln Lys Ser Leu Phe Asp Thr Thr Pro Phe Lys Ile Ala Met Val
        195                 200                 205

Gly Arg Tyr Ser Asn Glu Lys Asn Gln Ser Val Leu Ile Lys Ala Val
    210                 215                 220

Ala Leu Ser Lys Tyr Lys Gln Asp Ile Val Leu Leu Leu Lys Gly Lys
225                 230                 235                 240

Gly Pro Asp Glu Lys Lys Ile Lys Leu Leu Ala Gln Lys Leu Gly Val
                245                 250                 255

Lys Ala Glu Phe Gly Phe Val Asn Ser Asn Glu Leu Leu Glu Ile Leu
            260                 265                 270

Lys Thr Cys Thr Leu Tyr Val His Ala Ala Asn Val Glu Ser Glu Ala
        275                 280                 285

Ile Ala Cys Leu Glu Ala Ile Ser Val Gly Ile Val Pro Val Ile Ala
    290                 295                 300

Asn Ser Pro Leu Ser Ala Thr Arg Gln Phe Ala Leu Asp Glu Arg Ser
305                 310                 315                 320

Leu Phe Glu Pro Asn Asn Ala Lys Asp Leu Ser Ala Lys Ile Asp Trp
                325                 330                 335

Trp Leu Glu Asn Lys Leu Glu Arg Glu Arg Met Gln Asn Glu Tyr Ala
            340                 345                 350

Lys Ser Ala Leu Asn Tyr Thr Leu Glu Asn Ser Val Ile Gln Ile Glu
        355                 360                 365

Lys Val Tyr Glu Glu Ala Ile Arg Asp Phe Lys Asn Asn Pro His Leu
    370                 375                 380

Phe Lys Thr Leu Ser
385

<210>  3
<211>  1170
<212>  DNA
<213>  Helicobacter pylori strain J99

<220>
<221>  CDS
<222>  (1)..(1170)

<400>  3
atg gtt att gtt tta gtc gtg gat agc ttt aaa gac acc agt aat ggc      48
Met Val Ile Val Leu Val Val Asp Ser Phe Lys Asp Thr Ser Asn Gly
1               5                   10                  15

act tct atg aca gcg ttt cgt ttt ttt gaa gcg ctg aaa aaa aga ggg      96
Thr Ser Met Thr Ala Phe Arg Phe Phe Glu Ala Leu Lys Lys Arg Gly
            20                  25                  30

cat gcg atg aga gtg gtc gcc cct cat gtg gat aat tta ggg agt gaa      144
His Ala Met Arg Val Val Ala Pro His Val Asp Asn Leu Gly Ser Glu
        35                  40                  45

gaa gag ggg tat tac aac ctt aaa gag cgc tat atc ccc cta gtt aca      192
Glu Glu Gly Tyr Tyr Asn Leu Lys Glu Arg Tyr Ile Pro Leu Val Thr
    50                  55                  60

gaa att tca cac aag caa cac att ctt ttt gcc aaa ccg gat gaa aaa      240
Glu Ile Ser His Lys Gln His Ile Leu Phe Ala Lys Pro Asp Glu Lys
65                  70                  75                  80

att cta cga aag gct ttt aag gga gcg gat atg atc cat act tac ttg      288
Ile Leu Arg Lys Ala Phe Lys Gly Ala Asp Met Ile His Thr Tyr Leu
                85                  90                  95

cct ttt ttg cta gaa aaa aca gcc gta aaa atc gcg cga gaa atg cga      336
Pro Phe Leu Leu Glu Lys Thr Ala Val Lys Ile Ala Arg Glu Met Arg
            100                 105                 110

gtg cct tat att ggc tct ttc cat tta cag cca gag cat att tct tat      384
Val Pro Tyr Ile Gly Ser Phe His Leu Gln Pro Glu His Ile Ser Tyr
        115                 120                 125

aac atg aaa ttg ggg caa ttt tct tgg cta aat acc atg ctt ttt tca 432
Asn Met Lys Leu Gly Gln Phe Ser Trp Leu Asn Thr Met Leu Phe Ser
    130                 135                 140

tgg ttt aaa tct tcg cat tac cgc tat atc cac cat atc cat tgc cca 480
Trp Phe Lys Ser Ser His Tyr Arg Tyr Ile His His Ile His Cys Pro
145                 150                 155                 160

tca aaa ttc att gta gaa gaa ttg gaa aaa tac aac tat gga gga aaa 528
Ser Lys Phe Ile Val Glu Glu Leu Glu Lys Tyr Asn Tyr Gly Gly Lys
                165                 170                 175

aaa tac gct atc tct aac ggc ttt gat ccc atg ttt aag ttt gag cac 576
Lys Tyr Ala Ile Ser Asn Gly Phe Asp Pro Met Phe Lys Phe Glu His
            180                 185                 190

ccg caa aaa agc ctt ttt gac acc acg ccc ttt aaa atc gct atg gta 624
Pro Gln Lys Ser Leu Phe Asp Thr Thr Pro Phe Lys Ile Ala Met Val
        195                 200                 205

ggg cgc tat tct aat gaa aaa aat caa agc gtt ctc att aaa gcg gtt 672
Gly Arg Tyr Ser Asn Glu Lys Asn Gln Ser Val Leu Ile Lys Ala Val
    210                 215                 220

gct tta agc cga tac aaa caa gac att gta tta tta ctc aaa ggc aag 720
Ala Leu Ser Arg Tyr Lys Gln Asp Ile Val Leu Leu Leu Lys Gly Lys
225                 230                 235                 240

ggg cct gat gag aaa aaa atc aaa ctt cta gcc caa aaa cta ggc gta 768
Gly Pro Asp Glu Lys Lys Ile Lys Leu Leu Ala Gln Lys Leu Gly Val
                245                 250                 255

aaa acg gag ttt ggg ttt gtc aat tcc cat gaa ttg tta gag att tta 816
Lys Thr Glu Phe Gly Phe Val Asn Ser His Glu Leu Leu Glu Ile Leu
            260                 265                 270

aaa act tgc acc ctc tat gcg cac acg gcc aat gtg gaa agc gaa gcg 864
Lys Thr Cys Thr Leu Tyr Ala His Thr Ala Asn Val Glu Ser Glu Ala
        275                 280                 285

att gcg tgt tta gaa gcc att agc gtg ggg att gtg cct gtt atc gct 912
Ile Ala Cys Leu Glu Ala Ile Ser Val Gly Ile Val Pro Val Ile Ala
    290                 295                 300

aat agc cct tta agc gcg acc agg caa ttc gcg cta gat gaa cga tcg 960
Asn Ser Pro Leu Ser Ala Thr Arg Gln Phe Ala Leu Asp Glu Arg Ser
305                 310                 315                 320

tta ttt gag cct aat aac gct aaa gat ttg agc gct aaa ata gac tgg 1008
Leu Phe Glu Pro Asn Asn Ala Lys Asp Leu Ser Ala Lys Ile Asp Trp
                325                 330                 335

tgg tta gaa aac aaa ctt gaa aga gaa aga atg caa aac gaa tac gct 1056
Trp Leu Glu Asn Lys Leu Glu Arg Glu Arg Met Gln Asn Glu Tyr Ala
            340                 345                 350

aaa agc gct tta aac tac act tta gaa aat tca gtc att caa att gaa 1104
Lys Ser Ala Leu Asn Tyr Thr Leu Glu Asn Ser Val Ile Gln Ile Glu
        355                 360                 365

aaa gtt tat gaa gaa gcg atc aaa gat ttt aaa aac aac ccc aac ctc 1152
Lys Val Tyr Glu Glu Ala Ile Lys Asp Phe Lys Asn Asn Pro Asn Leu
    370                 375                 380

ttt aaa acc tta tcg taa 1170
Phe Lys Thr Leu Ser
385

<210>  4
<211>  389
<212>  PRT
<213>  Helicobacter pylori strain J99

<400>  4

Met Val Ile Val Leu Val Val Asp Ser Phe Lys Asp Thr Ser Asn Gly
1               5                   10                  15

Thr Ser Met Thr Ala Phe Arg Phe Phe Glu Ala Leu Lys Lys Arg Gly
            20                  25                  30

His Ala Met Arg Val Val Ala Pro His Val Asp Asn Leu Gly Ser Glu
        35                  40                  45

Glu Glu Gly Tyr Tyr Asn Leu Lys Glu Arg Tyr Ile Pro Leu Val Thr
    50                  55                  60

Glu Ile Ser His Lys Gln His Ile Leu Phe Ala Lys Pro Asp Glu Lys
65                  70                  75                  80

Ile Leu Arg Lys Ala Phe Lys Gly Ala Asp Met Ile His Thr Tyr Leu
                85                  90                  95

Pro Phe Leu Leu Glu Lys Thr Ala Val Lys Ile Ala Arg Glu Met Arg
            100                 105                 110

Val Pro Tyr Ile Gly Ser Phe His Leu Gln Pro Glu His Ile Ser Tyr
        115                 120                 125

Asn Met Lys Leu Gly Gln Phe Ser Trp Leu Asn Thr Met Leu Phe Ser
    130                 135                 140

Trp Phe Lys Ser Ser His Tyr Arg Tyr Ile His His Ile His Cys Pro
145                 150                 155                 160

Ser Lys Phe Ile Val Glu Glu Leu Glu Lys Tyr Asn Tyr Gly Gly Lys
                165                 170                 175

Lys Tyr Ala Ile Ser Asn Gly Phe Asp Pro Met Phe Lys Phe Glu His
            180                 185                 190

Pro Gln Lys Ser Leu Phe Asp Thr Thr Pro Phe Lys Ile Ala Met Val
        195                 200                 205

Gly Arg Tyr Ser Asn Glu Lys Asn Gln Ser Val Leu Ile Lys Ala Val
    210                 215                 220

Ala Leu Ser Arg Tyr Lys Gln Asp Ile Val Leu Leu Leu Lys Gly Lys
225                 230                 235                 240

Gly Pro Asp Glu Lys Lys Ile Lys Leu Leu Ala Gln Lys Leu Gly Val
                245                 250                 255

Lys Thr Glu Phe Gly Phe Val Asn Ser His Glu Leu Leu Glu Ile Leu
            260                 265                 270

Lys Thr Cys Thr Leu Tyr Ala His Thr Ala Asn Val Glu Ser Glu Ala
        275                 280                 285

Ile Ala Cys Leu Glu Ala Ile Ser Val Gly Ile Val Pro Val Ile Ala
    290                 295                 300

Asn Ser Pro Leu Ser Ala Thr Arg Gln Phe Ala Leu Asp Glu Arg Ser
305                 310                 315                 320

Leu Phe Glu Pro Asn Asn Ala Lys Asp Leu Ser Ala Lys Ile Asp Trp
                325                 330                 335

Trp Leu Glu Asn Lys Leu Glu Arg Glu Arg Met Gln Asn Glu Tyr Ala
            340                 345                 350

Lys Ser Ala Leu Asn Tyr Thr Leu Glu Asn Ser Val Ile Gln Ile Glu
        355                 360                 365

Lys Val Tyr Glu Glu Ala Ile Lys Asp Phe Lys Asn Asn Pro Asn Leu
    370                 375                 380

Phe Lys Thr Leu Ser
385

<210> 5
<211> 1179
<212> DNA
<213> Helicobacter hepaticus ATCC 51449

<220>
<221> CDS
<222> (1)..(1179)

<400> 5
atg att ata gcc ata gtc gta gat agt tat aaa gat aga agt aat ggc 48
Met Ile Ile Ala Ile Val Val Asp Ser Tyr Lys Asp Arg Ser Asn Gly
1               5                   10                  15

act tct atg aca gcc ttt cgc ttt gca aga gct ttg atc gaa aga gga 96
Thr Ser Met Thr Ala Phe Arg Phe Ala Arg Ala Leu Ile Glu Arg Gly
            20                  25                  30

cac tgt gtg cgc ata atc aca aca aat cct aca caa gaa gaa agc aat 144
His Cys Val Arg Ile Ile Thr Thr Asn Pro Thr Gln Glu Glu Ser Asn
        35                  40                  45

gaa caa agt agc ggt gaa gag ttt tat ttt gtc aaa gaa aga tat att 192
Glu Gln Ser Ser Gly Glu Glu Phe Tyr Phe Val Lys Glu Arg Tyr Ile
    50                  55                  60

cca atc gtt aca gaa atc tct cat aaa caa cat atg att ttt ggt tca 240
Pro Ile Val Thr Glu Ile Ser His Lys Gln His Met Ile Phe Gly Ser
65                  70                  75                  80

cca gat tct cac att tta gaa tct gcc ctt aaa ggt tgt gat att gca 288
Pro Asp Ser His Ile Leu Glu Ser Ala Leu Lys Gly Cys Asp Ile Ala
                85                  90                  95

cat ttt tat tta cct ttt gca tta gaa att gca ggt gtt cat ttg tgc 336
His Phe Tyr Leu Pro Phe Ala Leu Glu Ile Ala Gly Val His Leu Cys
            100                 105                 110

aga aag atg aag att ccc tat ata agt gct ttt cat ctc caa cct caa 384
Arg Lys Met Lys Ile Pro Tyr Ile Ser Ala Phe His Leu Gln Pro Gln
        115                 120                 125

cat atc agt tat aat atg aat atg aac ttt aaa tgg ttt aat gct tat 432
His Ile Ser Tyr Asn Met Asn Met Asn Phe Lys Trp Phe Asn Ala Tyr
    130                 135                 140

ctt ttt agg aga ttc tat aaa gcc ttt tat cgt tac aca cat cat atc 480
Leu Phe Arg Arg Phe Tyr Lys Ala Phe Tyr Arg Tyr Thr His His Ile
145                 150                 155                 160

cac tgc cca agt cag ctt atg gaa gaa gaa ata caa aag gca ggg tat 528
His Cys Pro Ser Gln Leu Met Glu Glu Glu Ile Gln Lys Ala Gly Tyr
                165                 170                 175

ggt ggg aaa aag ttt gtt ata agc aat gga ttc caa att aat aga gcg 576
Gly Gly Lys Lys Phe Val Ile Ser Asn Gly Phe Gln Ile Asn Arg Ala
            180                 185                 190

caa tct tgt gtg aag cca ttt gat gat aca ttt ttt cat att att tca 624
Gln Ser Cys Val Lys Pro Phe Asp Asp Thr Phe Phe His Ile Ile Ser
        195                 200                 205

gtg gga aga ttc tct aaa gaa aag cgg cag gat att ttg ata aaa gct 672
Val Gly Arg Phe Ser Lys Glu Lys Arg Gln Asp Ile Leu Ile Lys Ala
    210                 215                 220

att act caa agt aaa tat gca aac aat atc aaa tta cat cta cac gga 720
Ile Thr Gln Ser Lys Tyr Ala Asn Asn Ile Lys Leu His Leu His Gly
225                 230                 235                 240

att gga cca aaa gag caa tat ctg cgc aaa ctc tgc gat aaa ctt tta 768
Ile Gly Pro Lys Glu Gln Tyr Leu Arg Lys Leu Cys Asp Lys Leu Leu
                245                 250                 255

atc aat ccg cct gaa ttt ggt ttt att gat aat aat ata ctc att gaa 816
Ile Asn Pro Pro Glu Phe Gly Phe Ile Asp Asn Asn Ile Leu Ile Glu
            260                 265                 270

aaa ctt aaa tgt tgc cat ttg tat gtg cat tct gcg gaa gtt gaa agc 864
Lys Leu Lys Cys Cys His Leu Tyr Val His Ser Ala Glu Val Glu Ser
        275                 280                 285

gag gct att tct tgc cta gag gca gtt tct att gga tta gtt cct gtt 912
Glu Ala Ile Ser Cys Leu Glu Ala Val Ser Ile Gly Leu Val Pro Val
    290                 295                 300

att gct gat tct aaa ata agt gct act aca caa ttt gct ctt gat gaa 960
Ile Ala Asp Ser Lys Ile Ser Ala Thr Thr Gln Phe Ala Leu Asp Glu
305                 310                 315                 320

aga agc tta ttt aaa aca aat aat ata gcg gat tta agc gca aaa att 1008
Arg Ser Leu Phe Lys Thr Asn Asn Ile Ala Asp Leu Ser Ala Lys Ile
                325                 330                 335

gac tat tgg gta gaa cac tct aaa gaa cga gag cag atg aaa gag caa 1056
Asp Tyr Trp Val Glu His Ser Lys Glu Arg Glu Gln Met Lys Glu Gln
            340                 345                 350

tat gcc aaa agt gca tca aaa tat gca ctt gag aaa agt att gat aaa 1104

Tyr Ala Lys Ser Ala Ser Lys Tyr Ala Leu Glu Lys Ser Ile Asp Lys
        355                 360                 365

atc att aat atg tat gaa gaa gct atc acc gat ttt acg aca aat cct 1152
Ile Ile Asn Met Tyr Glu Glu Ala Ile Thr Asp Phe Thr Thr Asn Pro
    370                 375                 380

ata tta ttt aaa gcc att aac gct tga 1179
Ile Leu Phe Lys Ala Ile Asn Ala
385                 390

<210> 6
<211> 392
<212> PRT
<213> Helicobacter hepaticus ATCC 51449

<400> 6

Met Ile Ile Ala Ile Val Val Asp Ser Tyr Lys Asp Arg Ser Asn Gly
1               5                   10                  15

Thr Ser Met Thr Ala Phe Arg Phe Ala Arg Ala Leu Ile Glu Arg Gly
            20                  25                  30

His Cys Val Arg Ile Ile Thr Thr Asn Pro Thr Gln Glu Glu Ser Asn
        35                  40                  45

Glu Gln Ser Ser Gly Glu Glu Phe Tyr Phe Val Lys Glu Arg Tyr Ile
    50                  55                  60

Pro Ile Val Thr Glu Ile Ser His Lys Gln His Met Ile Phe Gly Ser
65                  70                  75                  80

Pro Asp Ser His Ile Leu Glu Ser Ala Leu Lys Gly Cys Asp Ile Ala
                85                  90                  95

His Phe Tyr Leu Pro Phe Ala Leu Glu Ile Ala Gly Val His Leu Cys
            100                 105                 110

Arg Lys Met Lys Ile Pro Tyr Ile Ser Ala Phe His Leu Gln Pro Gln
        115                 120                 125

His Ile Ser Tyr Asn Met Asn Met Asn Phe Lys Trp Phe Asn Ala Tyr
    130                 135                 140

Leu Phe Arg Arg Phe Tyr Lys Ala Phe Tyr Arg Tyr Thr His His Ile
145                 150                 155                 160

His Cys Pro Ser Gln Leu Met Glu Glu Glu Ile Gln Lys Ala Gly Tyr
                165                 170                 175

Gly Gly Lys Lys Phe Val Ile Ser Asn Gly Phe Gln Ile Asn Arg Ala
            180                 185                 190

Gln Ser Cys Val Lys Pro Phe Asp Asp Thr Phe Phe His Ile Ile Ser
        195                 200                 205

Val Gly Arg Phe Ser Lys Glu Lys Arg Gln Asp Ile Leu Ile Lys Ala
    210                 215                 220

Ile Thr Gln Ser Lys Tyr Ala Asn Asn Ile Lys Leu His Leu His Gly
225                 230                 235                 240

Ile Gly Pro Lys Glu Gln Tyr Leu Arg Lys Leu Cys Asp Lys Leu Leu
                245                 250                 255

Ile Asn Pro Pro Glu Phe Gly Phe Ile Asp Asn Asn Ile Leu Ile Glu
            260                 265                 270

Lys Leu Lys Cys Cys His Leu Tyr Val His Ser Ala Glu Val Glu Ser
        275                 280                 285

Glu Ala Ile Ser Cys Leu Glu Ala Val Ser Ile Gly Leu Val Pro Val
    290                 295                 300

Ile Ala Asp Ser Lys Ile Ser Ala Thr Thr Gln Phe Ala Leu Asp Glu
305                 310                 315                 320

Arg Ser Leu Phe Lys Thr Asn Asn Ile Ala Asp Leu Ser Ala Lys Ile
                325                 330                 335

Asp Tyr Trp Val Glu His Ser Lys Glu Arg Glu Gln Met Lys Glu Gln
            340                 345                 350

Tyr Ala Lys Ser Ala Ser Lys Tyr Ala Leu Glu Lys Ser Ile Asp Lys
        355                 360                 365

Ile Ile Asn Met Tyr Glu Glu Ala Ile Thr Asp Phe Thr Thr Asn Pro
    370                 375                 380

Ile Leu Phe Lys Ala Ile Asn Ala
385                 390

<210>  7
<211>  39
<212>  DNA
<213>  Artificial

<220>
<223>  HP0421 forward primer

<400>  7
gtacggatcc accatggtta ttgttttagt cgtggatag                              39

<210>  8
<211>  46
<212>  DNA
<213>  Artificial

<220>
<223>  HP0421 reverse primer

<400>  8
acgtggatcc cgtgcggccg ctgataaggt tttaaagaga tggggg                    46

<210>  9
<211>  441
<212>  PRT
<213>  Streptococcus pneumoniae

<400>  9

Met Arg Ile Gly Leu Phe Thr Asp Thr Tyr Phe Pro Gln Val Ser Gly
1               5                   10                  15

Val Ala Thr Ser Ile Arg Thr Leu Lys Thr Glu Leu Glu Lys Gln Gly
            20                  25                  30

His Ala Val Phe Ile Phe Thr Thr Thr Asp Lys Asp Val Asn Arg Tyr
        35                  40                  45

Glu Asp Trp Gln Ile Ile Arg Ile Pro Ser Val Pro Phe Phe Ala Phe
    50                  55                  60

Lys Asp Arg Arg Phe Ala Tyr Arg Gly Phe Ser Lys Ala Leu Glu Ile
65                  70                  75                  80

Ala Lys Gln Tyr Gln Leu Asp Ile Ile His Thr Gln Thr Glu Phe Ser
                85                  90                  95

Leu Gly Leu Leu Gly Ile Trp Ile Ala Arg Glu Leu Lys Ile Pro Val
            100                 105                 110

Ile His Thr Tyr His Thr Gln Tyr Glu Asp Tyr Val His Tyr Ile Ala
        115                 120                 125

Lys Gly Met Leu Ile Arg Pro Ser Met Val Lys Tyr Leu Val Arg Gly
    130                 135                 140

Phe Leu His Asp Val Asp Gly Val Ile Cys Pro Ser Glu Ile Val Arg
145                 150                 155                 160

Asp Leu Leu Ser Asp Tyr Lys Val Lys Val Glu Lys Arg Val Ile Pro
                165                 170                 175

Thr Gly Ile Glu Leu Ala Lys Phe Glu Arg Pro Glu Ile Lys Gln Glu
            180                 185                 190

Asn Leu Lys Glu Leu Arg Ser Lys Leu Gly Ile Gln Asp Gly Glu Lys
        195                 200                 205

Thr Leu Leu Ser Leu Ser Arg Ile Ser Tyr Glu Lys Asn Ile Gln Ala
    210                 215                 220

Val Leu Val Ala Phe Ala Asp Val Leu Lys Glu Glu Asp Lys Val Lys

225                 230                 235                 240

Leu Val Val Ala Gly Asp Gly Pro Tyr Leu Asn Asp Leu Lys Glu Gln
                245                 250                 255

Ala Gln Asn Leu Glu Ile Gln Asp Ser Val Ile Phe Thr Gly Met Ile
            260                 265                 270

Ala Pro Ser Glu Thr Ala Leu Tyr Tyr Lys Ala Ala Asp Phe Phe Ile
        275                 280                 285

Ser Ala Ser Thr Ser Glu Thr Gln Gly Leu Thr Tyr Leu Glu Ser Leu
    290                 295                 300

Ala Ser Gly Thr Pro Val Ile Ala His Gly Asn Pro Tyr Leu Asn Asn
305                 310                 315                 320

Leu Ile Ser Asp Lys Met Phe Gly Ala Leu Tyr Tyr Gly Glu His Asp
                325                 330                 335

Leu Ala Gly Ala Ile Leu Glu Ala Leu Ile Ala Thr Pro Asp Met Asn
            340                 345                 350

Glu His Thr Leu Ser Glu Lys Leu Tyr Glu Ile Ser Ala Glu Asn Phe
        355                 360                 365

Gly Lys Arg Val His Glu Phe Tyr Leu Asp Ala Ile Ile Ser Asn Asn
    370                 375                 380

Phe Gln Lys Asp Leu Ala Lys Asp Asp Thr Val Ser Gln Arg Ile Phe
385                 390                 395                 400

Lys Thr Val Leu Tyr Leu Pro Gln Gln Val Val Ala Val Pro Val Lys
                405                 410                 415

Gly Ser Arg Arg Met Leu Lys Ala Ser Lys Thr Gln Leu Ile Ser Met
            420                 425                 430

Arg Asp Tyr Trp Lys Asp His Glu Glu
        435                 440

<210>  10
<211>  398
<212>  PRT
<213>  Acholeplasma laidlawii

<400>  10

Met Arg Ile Gly Ile Phe Ser Glu Ala Tyr Leu Pro Leu Ile Ser Gly
1               5                   10                  15

Val Val Thr Ser Val Val Asn Leu Lys Glu Gly Leu Glu Ala Leu Gly
            20                  25                  30

His Glu Val Tyr Val Ile Thr Pro Ile Pro Ser Lys Asp Lys Phe Glu
        35                  40                  45

Asn Asp Pro Ser Val Ile Arg Ile Pro Gly Trp Val Ile Pro Arg Lys
    50                  55                  60

Ser Leu Lys Gly Phe Arg Leu Val Leu Phe Val Lys Arg Tyr Val Arg
65                  70                  75                  80

Lys Met Arg Lys Leu Lys Leu Asp Val Val His Ile His Thr Glu Phe
                85                  90                  95

Ser Met Gly Lys Leu Gly Leu Ala Val Ala Lys Lys Glu Arg Ile Pro
            100                 105                 110

Ser Val Tyr Thr Leu His Thr Ser Tyr Gln Asp Tyr Thr His Tyr Val
        115                 120                 125

Ser Lys Leu Leu Thr Arg Phe Ala Pro Asn Ala Ala Lys Lys Leu Ala
    130                 135                 140

Gly Lys Ile Asn Asn Gln Tyr Thr Lys Asn Cys His Met Thr Ile Val
145                 150                 155                 160

Pro Thr Lys Lys Ile Tyr Asp Lys Met Ile Arg Leu Lys His Asp Gly
                165                 170                 175

Glu Phe Thr Ile Ile Pro Ser Gly Ile Asn Leu Lys Pro Phe Tyr Lys
            180                 185                 190

Ser Ser Tyr Thr Ser Glu Gln Val Gln Ala Leu Lys Asp Lys Leu Gly
        195                 200                 205

Ile Arg Asn Asp Glu Phe Val Ala Ile Leu Val Ala Arg Ile Ala Lys
    210                 215                 220

Glu Lys Ser Ile Gly Asp Leu Val Glu Ala Phe Val Glu Phe Tyr Lys
225                 230                 235                 240

Ser Tyr Pro Asn Ser Arg Phe Ile Ile Ile Gly Asp Gly Pro Asp Lys
                245                 250                 255

Pro Val Leu Asp Lys Leu Ile Asp Ser Lys Lys Ala Ser Lys Tyr Ile
            260                 265                 270

Asn Thr Leu Gly Phe Val Lys Asn Ala Glu Val Gly Leu Tyr Tyr Gln
        275                 280                 285

Ile Ala Asp Val Phe Leu Asn Ala Ser Thr Thr Glu Thr Gln Gly Leu
    290                 295                 300

Thr Tyr Val Glu Ala Leu Ala Ala Ser Leu Pro Ile Ile Val Arg Tyr
305                 310                 315                 320

Asp Asp Val Phe Asp Ala Phe Val Glu Asp Gly Lys Asn Gly Ile Phe
                325                 330                 335

Phe Asn Lys Asn Glu Glu Leu Val Lys His Leu Ile His Ile Arg Gln
            340                 345                 350

Asn Pro Glu Ile Leu Gly Thr Leu Ser Lys Asn Ala Glu Ile Ser Thr
        355                 360                 365

Lys Pro Tyr Ala Lys Glu Val Tyr Ala Lys Ser Cys Glu Thr Leu Tyr
    370                 375                 380

Leu Asp Leu Ile Asp Lys Asn Asn Lys Lys Leu Asn Lys Lys
385                 390                 395

<210>  11
<211>  406
<212>  PRT
<213>  Thermotoga maritima

<400>  11
   Met Asn Ile Ala Met Phe Ser Asp Thr Tyr Ala Pro Gln Ile Asn Gly
   1               5                   10                  15
   Val Ala Thr Ser Ile Arg Val Tyr Lys Lys Lys Leu Thr Glu Arg Gly
               20                  25                  30
   His Lys Val Val Val Val Ala Pro Ser Ala Pro Glu Glu Glu Lys Asp
           35                  40                  45
   Val Phe Val Val Arg Ser Ile Pro Phe Pro Phe Glu Pro Gln His Arg
       50                  55                  60
   Ile Ser Ile Ala Ser Thr Lys Asn Ile Leu Glu Phe Met Arg Glu Asn
   65                  70                  75                  80
   Asn Val Gln Ile Ile His Ser His Ser Pro Phe Phe Ile Gly Phe Lys
                   85                  90                  95
   Ala Leu Arg Val Gln Glu Glu Met Gly Leu Pro His Val His Thr Tyr
               100                 105                 110
   His Thr Leu Leu Pro Glu Tyr Arg His Tyr Ile Pro Lys Pro Phe Thr
           115                 120                 125
   Pro Pro Lys Arg Leu Val Glu His Phe Ser Ala Trp Phe Cys Asn Met
       130                 135                 140
   Thr Asn Val Val Ile Ala Pro Thr Glu Asp Ile Lys Arg Glu Leu Glu
   145                 150                 155                 160
   Ser Tyr Gly Val Lys Arg Pro Ile Glu Val Leu Pro Thr Gly Ile Glu
                   165                 170                 175
   Val Glu Lys Phe Glu Val Glu Ala Pro Glu Glu Leu Lys Arg Lys Trp
               180                 185                 190
   Asn Pro Glu Gly Lys Lys Val Val Leu Tyr Ala Gly Arg Ile Ala Lys
           195                 200                 205
   Glu Lys Asn Leu Asp Phe Leu Leu Arg Val Phe Glu Ser Leu Asn Ala
       210                 215                 220
   Pro Gly Ile Ala Phe Ile Met Val Gly Asp Gly Pro Glu Arg Glu Glu
   225                 230                 235                 240
   Val Glu Glu Phe Ala Lys Glu Lys Gly Leu Asp Leu Lys Ile Thr Gly
                   245                 250                 255
   Phe Val Pro His Asp Glu Ile Pro Leu Tyr Tyr Lys Leu Gly Asp Val
               260                 265                 270
   Phe Val Phe Ala Ser Lys Thr Glu Thr Gln Gly Leu Val Leu Leu Glu
           275                 280                 285
   Ala Leu Ala Ser Gly Leu Pro Val Val Ala Leu Lys Trp Lys Gly Val
       290                 295                 300
   Lys Asp Val Leu Lys Asn Cys Glu Ala Ala Val Leu Ile Glu Glu Glu
   305                 310                 315                 320
   Asn Glu Arg Leu Phe Ala Glu Lys Ile Lys His Ile Leu Lys Asn Asp
                   325                 330                 335
   Arg Leu Arg Glu Glu Leu Ser Thr Lys Gly Arg Glu Phe Val Arg Lys
               340                 345                 350
   Glu Trp Ser Val Asp Arg Phe Val Gln Arg Leu Glu Glu Ile Tyr Thr
           355                 360                 365
   Arg Ala Ile Glu Glu Gly Pro Val Glu Ile Asn Thr Ser Leu Met Ile
       370                 375                 380
   Lys Glu Phe Val Lys Phe Glu Lys Leu Lys Glu Phe Phe Ser Lys Ile
   385                 390                 395                 400
   Glu Asp Arg Ile Trp Arg
                   405

<210>  12
<211>  402
<212>  PRT

<213>  Deinococcus radiodurans

<400>  12

Met Asn Pro Leu Arg Ile Gly Leu Phe Thr Asp Thr Phe Leu Pro Asp
1               5                   10                  15

Gln Asn Gly Ile Val Thr Ser Val Cys Leu Leu Ser Asp Glu Leu Arg
            20                  25                  30

Ala Leu Gly His Gln Val Asp Val Val Ala Pro Arg Phe Pro Glu His
        35                  40                  45

Leu Asp Arg Arg Pro Asp Val Arg Arg Val Ala Ser Val Arg Tyr Pro
    50                  55                  60

Leu Leu Pro Thr Tyr Arg Leu Ala Trp Pro Gly Arg Arg Ser Phe Glu
65                  70                  75                  80

Gln Arg Tyr Asp Leu Ile His Thr His Thr Pro Leu Thr Leu Gly Leu
                85                  90                  95

Ala Gly Ala Arg Leu Ala Arg Lys Trp Gly Val Pro His Val Ala Thr
            100                 105                 110

Tyr His Thr His Leu Glu Ala Tyr Thr His Tyr Val Pro Gly Met Thr
        115                 120                 125

Gln Leu Asn Arg Ala Val His Phe Met Pro Lys Val Val Gly Arg Leu
    130                 135                 140

Tyr Gly Glu Ala Asp Ala Val Ile Thr Pro Thr Ala Gly Thr Leu Asp
145                 150                 155                 160

Thr Leu Arg Gln Ala Gly Ile Thr Asp Ala Val Val Ile Pro Thr Ser
                165                 170                 175

Ile Asp Pro Ala Val Leu Glu Ala Ala Pro Pro Ile Ala Ser Pro Trp
            180                 185                 190

Pro Pro Gly Thr Arg Arg Leu Leu Thr Val Gly Arg Leu Ala Arg Glu
        195                 200                 205

Lys Arg Phe Asp Leu Val Leu Asp Thr Leu Ala Asp Leu Pro Gly Ala
    210                 215                 220

His Leu Val Ile Leu Gly Glu Gly Pro Glu Arg Thr His Leu Glu Asp
225                 230                 235                 240

His Ala Gln Arg Leu Gly Val Ala Glu His Val Thr Phe Val Gly Val
                245                 250                 255

Arg Pro Trp Thr Glu Ile Gly Ala Tyr Tyr Arg Leu Ala Glu Leu Phe

            260                 265                 270

Val Phe Ala Ser Asp Thr Glu Thr Gln Gly Leu Val Leu Gln Glu Ala
        275                 280                 285

Gln Leu Met Gly Val Pro Val Val Ala Val Gly Ala Arg Gly Thr Leu
    290                 295                 300

Thr Gly Val Gln Ser Gly Tyr Ser Gly Tyr Leu Thr Ala Pro Gly Asp
305                 310                 315                 320

Val Pro Glu Leu Val Arg Arg Ala Arg Glu Leu Leu Glu Ser Pro Glu
                325                 330                 335

Gln Tyr Ala Arg Phe Ser Arg Gln Ala Arg Glu Phe Gly Gly Arg Thr
            340                 345                 350

Thr Pro Ala Gly Val Ala Arg Gln Val Leu Ala Val Tyr Ala Arg Thr
        355                 360                 365

Leu Gly Leu Pro Pro Glu Ser Pro Asp Asp Pro Arg Ala Asp Gly Leu
    370                 375                 380

Ser Ala Pro Ala Glu Ala Ala Gly Arg Pro Arg Asn Asn Pro Val Tyr
385                 390                 395                 400

Gly Arg

## Description

The present invention refers to inhibitors of cholesteryl-α-glucosyltransferase and the use thereof for the manufacture of a medicament for the prevention or/and treatment of *Helicobacter* infection or/and a *Helicobacter*-associated disease. Another subject is the screening method for novel inhibitors of cholesteryl-α-glucosyltransferases.

The Gram-negative pathogenic bacterium *Helicobacter pylori* colonizes the stomach of 50% of mankind and has probably infected humans since their origins. Although many who are infected develop 'technical' gastritis and inflammatory infiltrates in the stomach, they experience no symptoms and the infection with this mostly extra-cellular bacterium goes unnoticed in most cases. *H. pylori* has however been shown to be responsible for 71% of gastric carcinoma cases, which accounts for 10% of cancer deaths worldwide. *H. pylori* is acquired during childhood by intra-familial transmission and infection continues lifelong in the absence of antibiotic therapy. Recombination is frequent during transient colonization with multiple strains due to DNA transformation, facilitating the acquisition of antibiotic resistances. *Helicobacter pylori* infection is associated with a variety of gastric disorders including chronic gastritis, peptic ulcer disease, mucosa associated lymphatic tissue (MALT) lymphoma and gastric adenocarcinoma. The pathogenicity of the bacterium is determined by epidemiological influences as well as bacterial and host factors. Bacterial colonization of the gastric mucosa leads to development of a chronic inflammatory infiltrate, which is accompanied by enhanced release of inflammatory mediators, growth factors and reactive oxygen metabolites.

Antibiotic therapy against *H*. *pylori (Hp)* is effective, however only combined triple therapy (2 antibiotics plus a proton pump inhibitor) is successful at eradicating the bacteria. This therapy is relatively expensive and an effective prophylactic or therapeutic *H. pylori* vaccine would be another way to control disease. However, to this date, no effective vaccine is available or expected to available. Yet, the mouse model of *H. pylori* infection has been invaluable for improving our understanding of the immune response to this bacterium, and vaccination is effective in this model. Numerous studies have attempted to reveal the immunological basis of immunization, and as a result of studies using knock-out mice it is now clear that protection is MHC-II and IFNy dependant. IL-4 and IL-13 are not required. Furthermore, it has been reported that CD4+ T cell lines of Th2 or mixed type were able to confer protection after transfer to naïve recipients. Despite these significant advances, a clear link to an effector mechanism cannot yet be made.

Given that protection is dependent on CD4+ cells, it is reasonable to assume that the protective effect is mediated via the epithelial cells of the gastric mucosa. Two possible models for protection have been proposed- one where *H. pylori* specific CD4+ T cells secrete mediators (probably cytokines) which influence gastric epithelial cells to produce secretory mediators, or possibly altered mucins which alone or in combination make the local environment unfavorable for *H. pylori.* Another intriguing proposal extends this conclusion and proposes that *H. pylori* may also come in direct contact with activated phagocytes.

A hallmark of *H. pylori* colonization of the stomach is the induction of a strong pro-inflammatory response of the infected epithelium which initiates mucosal infiltration by neutrophils, monocytes and lymphocytes leading to gastritis. The continual interplay between *H. pylori* and host has been recently demonstrated by showing that vacuolating cytotoxin A (VacA) blocks T cell proliferation.

Cholesterol is a physiological constituent of membranes critical for their biophysical properties, but is stigmatized as mediating detrimental effects in obesity and cardiovascular disease.

The problem of the present invention are novel treatment regimens against chronic and acute *Helicobacter* infections. Since *H. pylori* is auxotrophic for cholesterol, we explored the assimilation of cholesterol by *H. pylori* upon infection. Cholesterol promotes phagocytosis of *H. pylori* by antigen-presenting cells such as macrophages and dendritic cells and enhances antigen-specific T cell responses. In contrast, intrinsic α-glucosylation of cholesterol abrogates phagocytosis of *H. pylori* and subsequent T cell activation. Here we identified HP0421 as the gene encoding for cholesteryl-α-glucosyltransferase, the enzyme responsible for cholesterol glucosylation. This enzyme catalyses the first step in the biosynthesis of four unusual glycolipids: cholesterol-α-glucoside, cholesterol-6-O-acyl-α-glucoside, cholesterol-6-O-phosphatidyl-α-glucoside, and cholesterol-6-O-lysophosphatidyl-α-glucoside. Generation of knockout mutants lacking a functional HP0421 gene corroborates the importance of cholesterol glucosides for escaping phagocytosis, T cell activation and bacterial clearance in vivo.

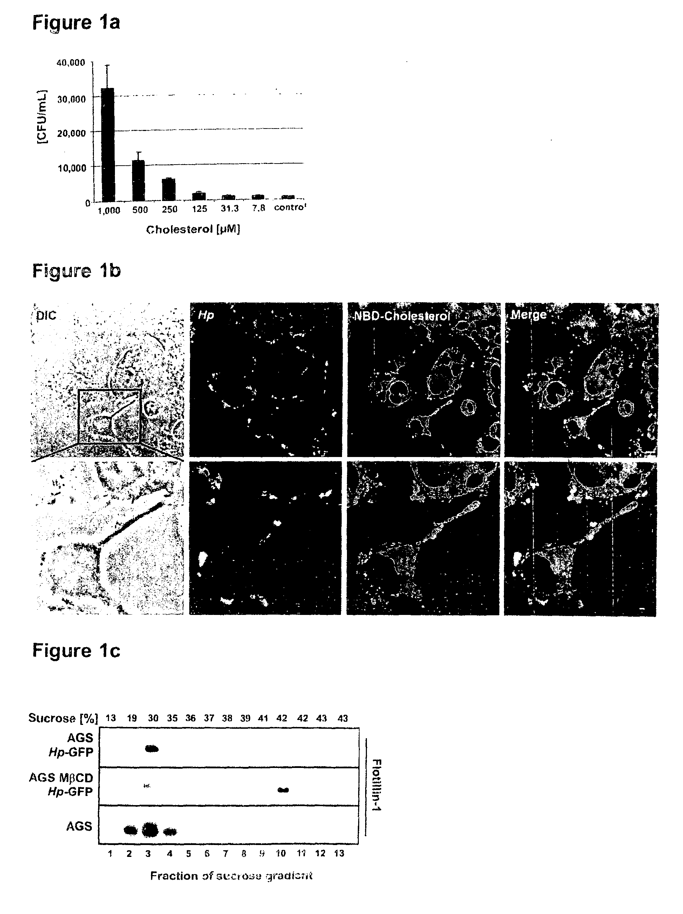

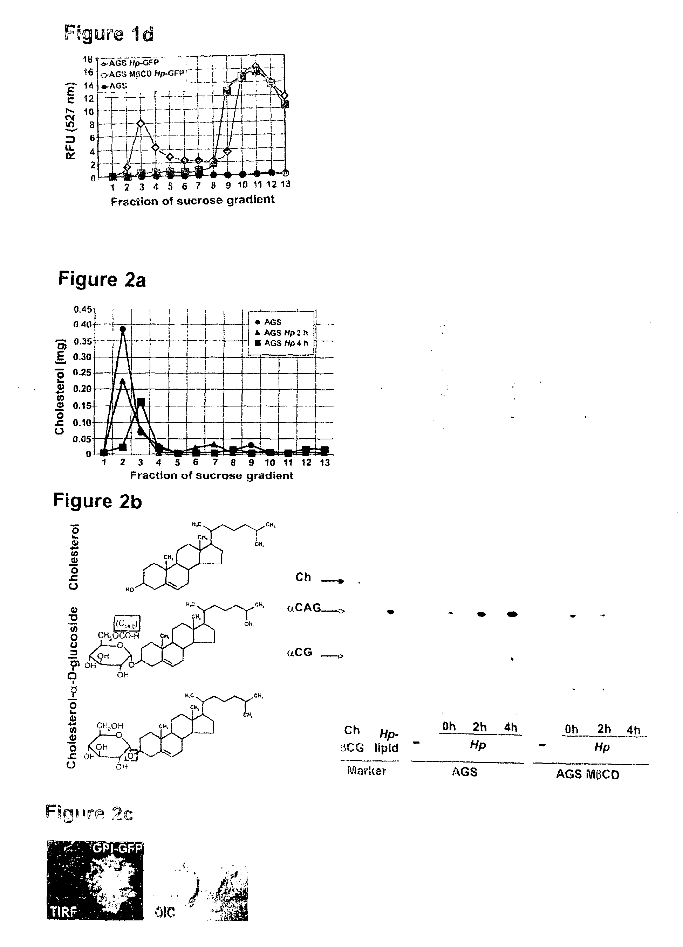

Surprisingly, we found that *H. pylori* follows a cholesterol gradient. Since *H*. *pylori* does not possess enzymes for de novo sterol synthesis, exogenous cholesterol is essential for growth of *H. pylori.* Therefore, we investigated whether *H. pylori* is capable of detecting cholesterol in the environment and is able to move towards the nutrient. For this purpose, we used a hermetic tube system, which consists of three tubes containing cholesterol, *H. pylori* or medium alone (Fig. S1a). All tubes were connected allowing *H. pylori* to move towards cholesterol or medium alone. Tube suspensions were collected at different time points, diluted and plated on agar medium for subsequent counting of colony forming units (CFU). Bacterial titres derived from the cholesterol-containing tube depended on the lipid concentration and incubation time (Fig. 1 a and Fig. S1b). Thus, *H. pylori* was able to detect cholesterol concentrations of 250 µM (Fig. 1a), i.e. 40 times lower than the average cholesterol level in human serum. However, *H. pylori* did not follow a gradient of other steroids or phospholipids indicating specific movement of the pathogen towards cholesterol (Fig. S1c). A non-motile *H. pylori* mutant (HpΔFlgE) migrated to neither the cholesterol-containing nor the control tube thereby excluding passive diffusion of bacteria. In conclusion, *H. pylori* senses cholesterol in the milieu and moves along increasing concentrations of the nutrient.

Surprisingly we found that *H. pylori* acquires cholesterol from the plasma membrane of epithelial cells. A significant proportion of *H. pylori* in the stomach resides at the epithelial lining. Thus, we investigated whether *H*. *pylori* absorbs cholesterol through direct contact with epithelial cells or assimilates the secreted lipid. Gastric cancer cells (AGS) were incubated with benz-oxa-diazol-labeled cholesterol (NBD-cholesterol). Subsequently, cells were intensively washed and infected with *H. pylori.* Confocal microscopy shows green-fluorescent cholesterol of AGS cells and Cy5/antiserum-labeled *H. pylori* (Hp, depicted in white) attached to the epithelial surface (Fig. 1 b). Colocalization (violet) of green-fluorescent cholesterol and bacteria (white) demonstrated uptake of the lipid by adherent *H. pylori.* Moreover, bacterial incorporation of cholesterol was time-dependent (Fig. S2). Additionally, *H. pylori* failed to incorporate green-fluorescent cholesterol from supernatant of NBD-cholesterol-loaded epithelial cells (Fig. S3).

Surprisingly we observed that *H. pylori* associates with cholesterol rich membrane domains. In order to assess a possible role of cholesterol-rich membrane regions such as lipid rafts in cholesterol acquisition, we lysed AGS cells infected with *H. pylori* expressing green fluorescent protein (GFP) in detergent-free buffer and fractionated the lysate using discontinuous sucrose gradient centrifugation (Song, K.S. et al., J. Biol. Chem. 271, 9690-9697 (1996)). Lipid raft fractions were identified by the presence of the marker protein flotillin-1 (Forster, L.J., De Hoog, C.L. & Mann M., Proc. Natl. Acad. Sci. U.S.A. 100, 5813-5818 (2003)) in light density fractions 2, 3 and 4 (Fig. 1 c). Fluorometric detection of GFP in the same fractions indicated interaction of *H. pylori* with lipid rafts (Fig. 1d). Pretreatment of AGS cells with the cholesterol depleting drug methyl-β-cyclodextrin (MβCD) abolished the fluorescence activity in fractions 2, 3 and 4 demonstrating that the eukaryotic source of cholesterol was critical for *H. pylori-*GFP association with these fractions (Fig. 1d). Additionally, confocal microscopy revealed colocalization of *H. pylori* (Hp, red) with GM1-ganglioside receptor which is characteristic for lipid rafts (Harder, T., Scheiffele, P., Verkade, P. & Simons, K., J. Cell. Biol. 141, 929-942 (1998)) and was labeled with cholera toxin B subunit-FITC (CT-B, green) (Fig. S4). Thus, *H. pylori* is closely associated with cholesterol-rich membrane domains of epithelial cells which may facilitate cholesterol acquisition.

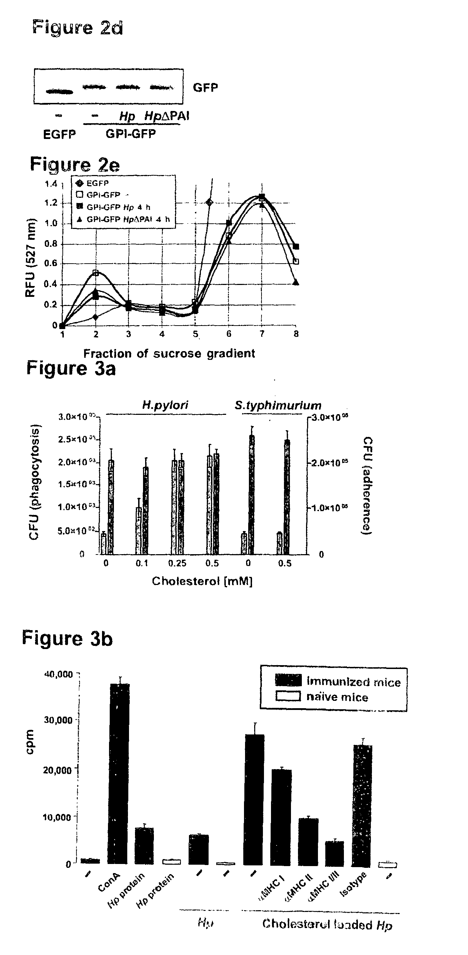

Next, we analysed the amount of cholesterol in *H*. pylori-containing sucrose gradient fractions by a colorimetric assay. Strikingly, the total amount of cholesterol in light membrane fractions 2 and 3, which harboured *H. pylori* after 2 or 4 h infection of AGS cells, was reduced over time (Fig. 2a). Despite cholesterol absorption by *H. pylori,* total cholesterol abundance in these fractions was expected to be constant since pathogen and cholesterol donor membranes of AGS cells were segregated in identical fractions. This apparent paradox could be due to modification of the hydroxyl group of cholesterol preventing its conversion by cholesterol oxidase used for enzymatic detection of cholesterol. Indeed, analysis of lipid extracts of *H. pylori*-infected cells by thin-layer chromatography (TLC) revealed an increase of glucosylated cholesterol derivatives after 2 or 4 h post infection (Fig. 2b). Mass spectrometry (MS) and nuclear magnetic resonance (NMR) analysis of these lipids identified cholesteryl-α-glucoside [cholesteryl-α-D-glucopyranoside (αCG)] and cholesteryl-acyl-glucoside [cholesteryl-6-O-tetradecanoyl-α-D-glucopyranoside (CAG)] that are unique in Helicobacter (Fig. S5). In order to determine whether cholesterol from prokaryotic or eukaryotic origin served as a substrate for bacterial cholesteryl-α-glucosyltransferase, the epithelial membrane of AGS cells was cholesterol-depleted using MβCD prior to infection with *H. pylori.* Subsequent lipid analysis of infected cells indicated that cholesterol-depletion prevented an increase in cholesteryl-α-glucosides (Fig. 2b). Thus, eukaryotic cholesterol was incorporated by *H. pylori* and coupled to D-glucose through α-glycosidic binding.

Surprisingly we observed that cholesterol facilitates phagocytosis of *H. pylori* and subsequent T cell activation. To investigate whether the cholesterol content of *H. pylori* has an impact on phagocytosis, *H. pylori* was incubated with varying cholesterol concentrations for 30 min, subsequently washed and used for infection of macrophages. Using a gentamicin protection assay (Fig. 3a) or differential immunofluorescence staining (Fig. S6), the amount of adherent or phagocytosed bacteria was determined by counting of CFU. Feeding *H. pylori* with cholesterol facilitated a dramatic increase in phagocytosis of bacteria by macrophages compared to untreated *H. pylori* as indicated by intracellular bacterial counts (Fig. 3a). In marked contrast, phagocytosis of Salmonella typhimurium, E. coli or Shigella flexneri was not affected by incubation of bacteria with cholesterol (Fig. 3a). Consequently, increased incorporation of cholesterol amplifies phagocytosis of *H. pylori* by antigen presenting cells (APCs).

We next examined the impact of cholesterol-promoted phagocytosis of *H*. *pylori* on T cell activation. To this end, T cells from spleen of C57BU6 mice immunized with *H. pylori* protein extract were added to *H*. pylori-infected APCs, macrophages or dendritic cells. Additionally, non-infected APCs were pulsed with *H. pylori* proteins and responder cells from non-immunized animals were included to determine antigen-specific T cell activation. Two days after stimulation, cells were pulsed with 3H-thymidine for subsequent measurement of T cell proliferation (Fig. 3b). T cells from non-immunized mice did not respond to protein-pulsed or infected APCs thus excluding unspecific T cell proliferation (Fig. 3b). However, *H. pylori* protein-pulsed APCs induced weak antigen-specific T cell activation of cells derived from immunized mice which was not amplified by addition of cholesterol (Fig. 3b). The antigen-specific T cell response was markedly increased when *H. pylori* was incubated with 1 mM cholesterol before infection compared to untreated bacteria (Fig. 3b). Strikingly, the cholesterol-mediated T cell response was comparable to polyclonal stimulation by the lectin concanavalin A. Addition of blocking antibodies to MHC-I or MHC-II before coculture with T cells abrogated the cholesterol-mediated increase of T cell activation compared to isotype controls (Fig. 3b). Thus, cholesterol induces increased MHC-restricted antigen presentation. Taken together, cholesterol promotes vigorous antigen-specific T cell activation upon *H. pylori* infection of APCs.

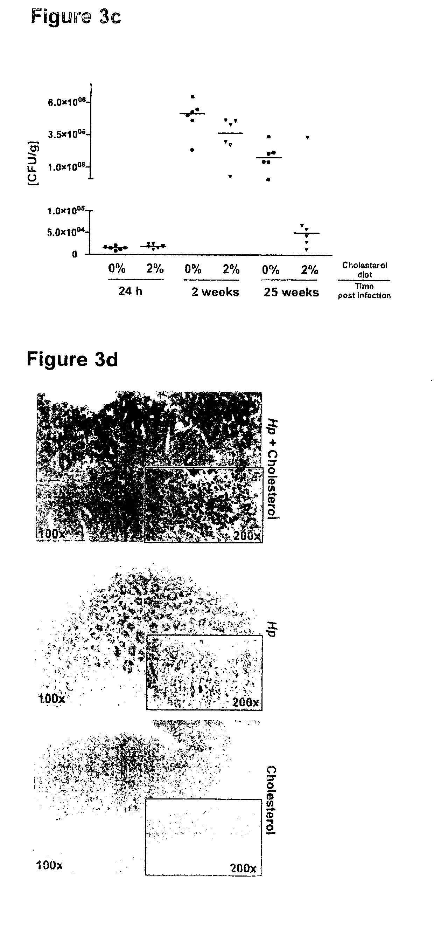

Interestingly, cholesterol mediates T cell dependent protection against *H. pylori* infection. In order to study the in vitro effect of cholesterol on *H. pylori* infection in vivo, we treated groups of C57BL/6 mice with a 2% or zero cholesterol diet initiated 14 d before infection. Subsequently, mice were orally infected with the mouse-adapted *H. pylori* strain Hp7622 or were left uninfected. 24 h post infection a uniformly moderate bacterial colonization was detected for all experimental groups by determining CFUs from gastric mucosa (Fig. 3c). Two weeks post infection, a minor decline in bacterial colonization was demonstrated for the high cholesterol diet group compared to mice not receiving extra lipid (Fig. 3c). The bacterial load was dramatically reduced in the presence of cholesterol 25 weeks after challenge, however (Fig. 3c). The reduction of gastric colonization by *H. pylori* by more than 20-fold upon high cholesterol diet was histomorphologically accompanied by a strong infiltration of neutrophils and lymphocytes as compared to infected animals receiving zero cholesterol diet (Fig. 3d). In contrast, no inflammation was observed in the non-infected high cholesterol group.

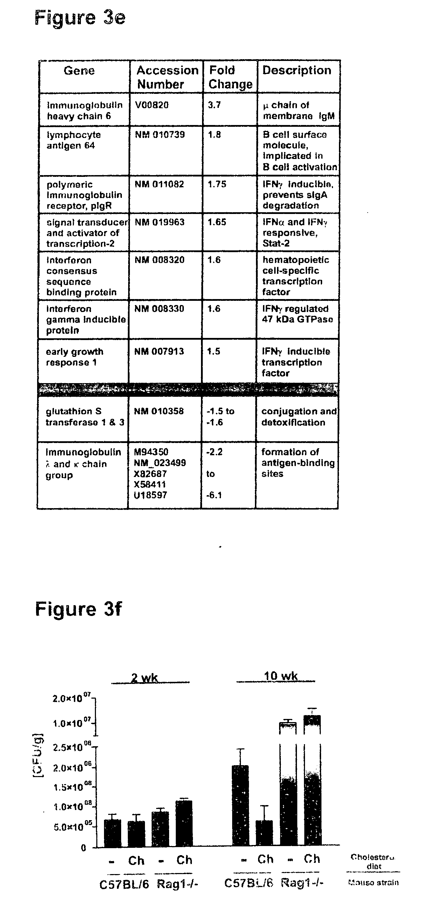

To examine whether the cholesterol-mediated reduction of bacteria in vivo was based on the presence of T cells, we treated RAG-deficient mice, which are devoid of T lymphocytes with high cholesterol diet before challenge with *H. pylori.* Analysis of the gastric bacterial burden 10 weeks after infection revealed a lack of cholesterol-mediated CFU reduction in the absence of T cells (RAG-KO) compared to wild-type mice (Fig. 3e). Taken together, the presence of cholesterol mediates potent T cell dependent reduction of *H. pylori* colonisation in the gastric mucosa.

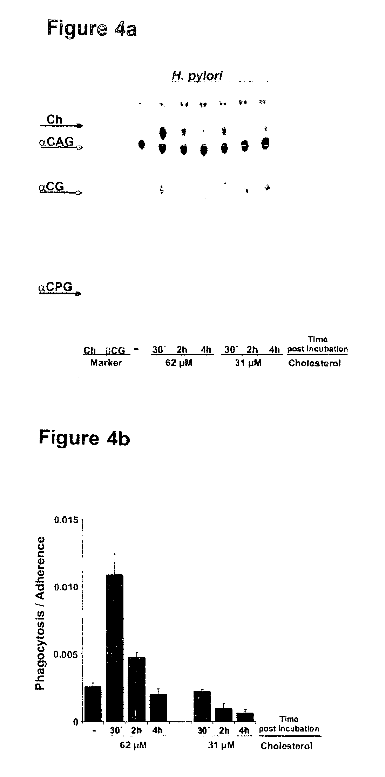

Unexpectedly, we observed that glycosylation of cholesterol by *H. pylori* mediates immune evasion. The total amount of lipids from *H. pylori* consists of 1.6% cholesterol and 25% cholesteryl glucosides (Haque et al., Acta Med. Okayama, 49: 205-211, 1995). Thus, we wanted to investigate the functional differences of cholesterol and cholesteryl glucosides in bacterial metabolism and induction of immune responses. For this purpose, *H. pylori* was pulsed with cholesterol for 30 min, subsequently washed, and then cultured in RPMI medium for 0, 2 or 4 h. Lipid analysis by TLC revealed that exogenously loaded cholesterol declined slowly over time, whereas the amount of cholesteryl glucosides was complementarily increased (Fig. 4a). At different time points, phagocytosis of cholesterol-loaded *H. pylori* was determined by gentamicin protection assay, in parallel to lipid analysis (Fig. 4b). Phagocytosis of *H*. *pylori* receiving a low load of cholesterol (31 mM) was not enhanced. In contrast, a higher load of cholesterol (62 mM) potently amplified uptake of *H. pylori* by macrophages at early time points (Fig. 4b). The extent of phagocytosis was reduced with increasing incubation time, consistent with the steady conversion of cholesterol into cholesteryl glucosides. Hence, a high cholesteryl glucoside/cholesterol ratio abrogates uptake of the pathogen by APCs and faciliates immune evasion.

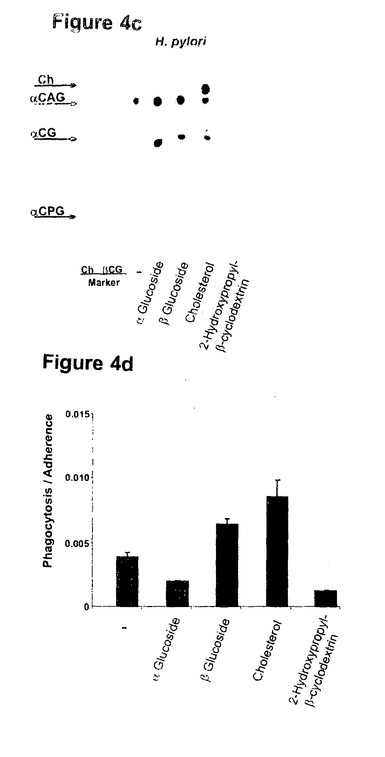

Following the assumption that cholesteryl glucosides modulate phagocytosis, *H. pylori* was exogenously loaded with both cholesteryl glucoside anomers and subsequently used to infect macrophages. TLC analysis of *H. pylori* used for infection revealed that cholesteryl-α-glucosides as well as cholesteryl-α-glucosides were acylated as indicated by an increase of CAG (Fig. 4c). Thus, both lipids were assimilated by *H. pylori* and were metabolically active. Subsequently, phagocytosis of cholesterol glucoside-loaded *H. pylori* by macrophages was investigated by determining intracellular bacterial counts (Fig. 4d). The addition of the β-glucoside induced pathogen uptake by APCs comparable to the effect of cholesterol. However, the presence of cholesteryl-α-glucoside abrogated engulfment of *H. pylori* by macrophages (Fig. 4d). Thus, cholesterol and its β-glucoside promote enhanced phagocytosis of *H. pylori* by APCs. In contrast, cholesteryl-α-glucosides protect *H. pylori* from phagocytosis and facilitate immune evasion.

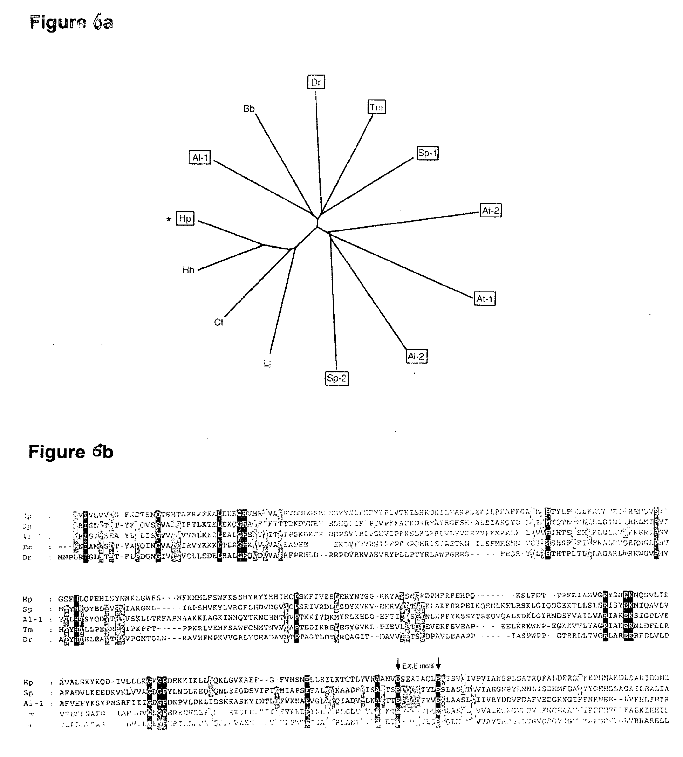

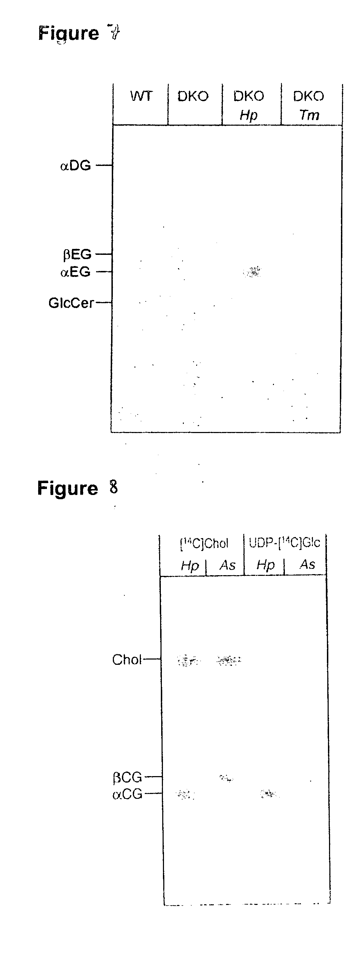

Based of the unexpected observations described above we aimed at elucidating the bacterial enzyme that mediates cholesterol glycosylation for subsequent genetic manipulation. The database at the Carbohydrate Active Enzyme Server (http://afmb.cnrs-mrs.fr/CAZY/index.html) (Campbell 1997) identified 21 hypothetical glycosyltransferase genes in the genome of *H*. *pylori* 26695 (23 in *H. pylori* J99), from which 5 (7 in *H. pylori* J99) represent retaining glycosyltransferases. Based on in silico analysis of these different *H. pylori* candidate genes, we found the gene product HP0421 to putatively form a cholesteryltransferase domain. The hypothetical protein HP0421 consists of 389 amino acids, is a member of the glycosyltransferase family 4 (GT4) and shares a conserved EX₇E motif with the other members of the family (Fig. 6). After cloning and overexpression of HP0421 in E. coli, testing of enzymatic activity revealed incorporation of radioactively labeled glucose into cholesterol by the gene product of HP0421, thereafter called cholesteryl-α-glucosyltransferase (Fig. 8). The substrate specifity is shown in Table I. The identified *H. pylori* cholesteryl-α-glucosyltransferase belongs to the glycosyltransferase family and shows similarities to some bacterial diacylglycerol-α-glucosyltransferases. Deletion of the HP0421 gene in *H. pylori* resulted in the loss of cholesterol-α-glucoside and all of its derivatives. Also, heterologous expression of HP0421 in the yeast Pichia pastoris led to the biosynthesis of ergosterol-α-glucoside as demonstrated by purification of the lipid and subsequent structural analysis by nuclear magnetic resonance spectroscopy and mass spectrometry (Fig. 7). In vitro enzyme assays were performed with cell-free homogenates obtained from cells of *H. pylori* or from transgenic Escherichia coli, which express HP0421. These assays revealed that the enzyme represents a membrane-bound, UDP-glucose-dependant cholesterol-α-glucosyltransferase.

To verify the lack of cholesterol glucosides in Δ421 *H. pylori* mutants, we performed lipid extraction from bacterial lysates followed by TLC analysis.

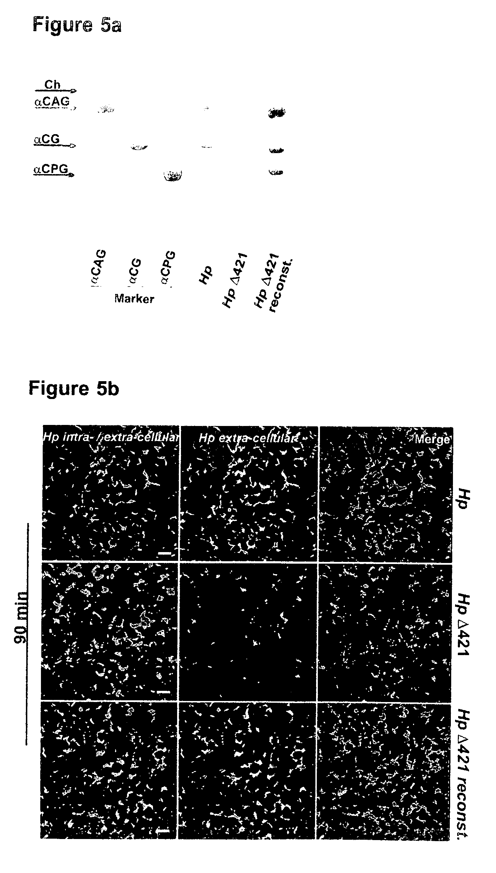

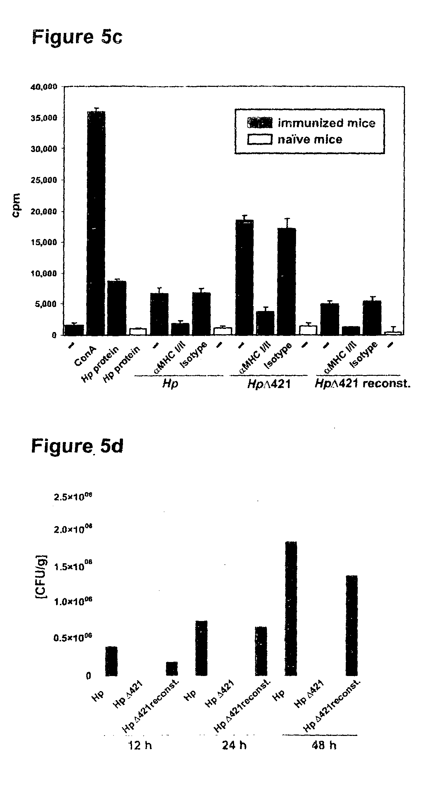

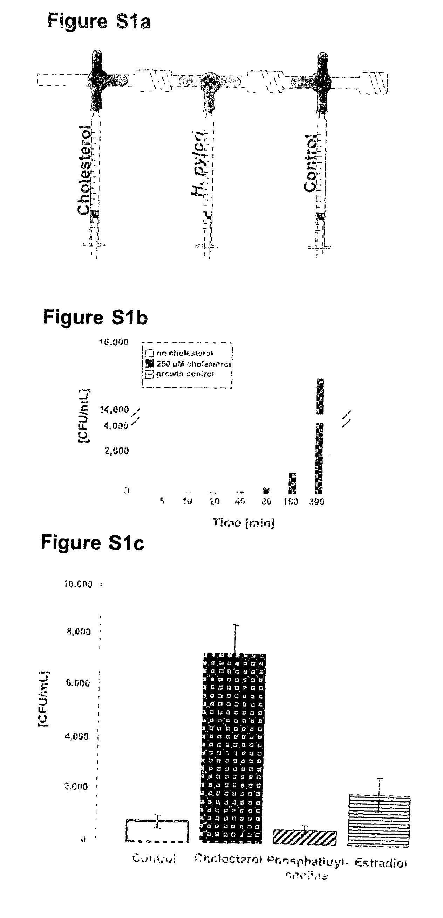

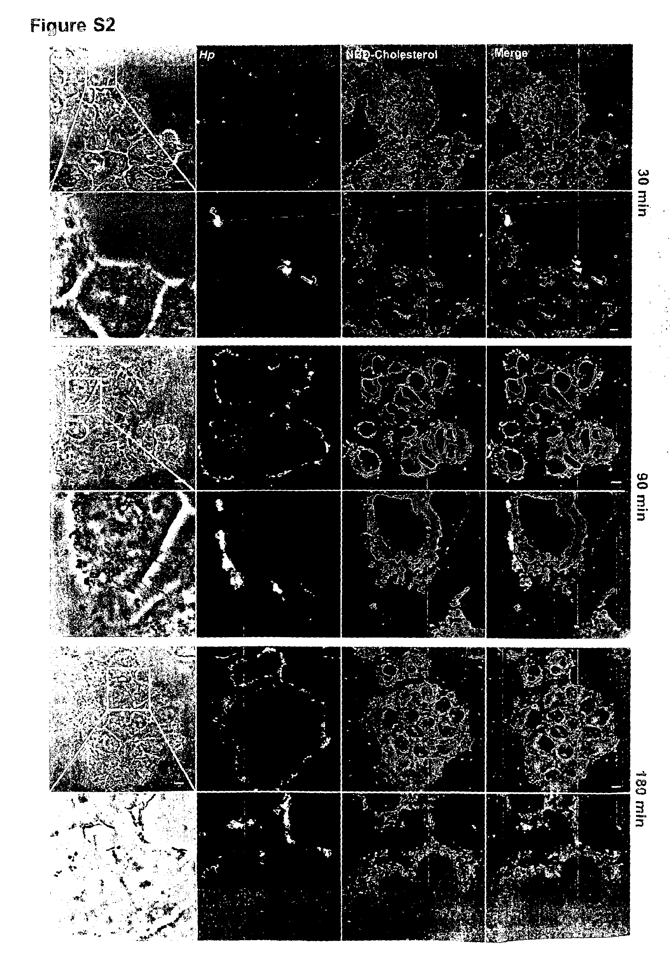

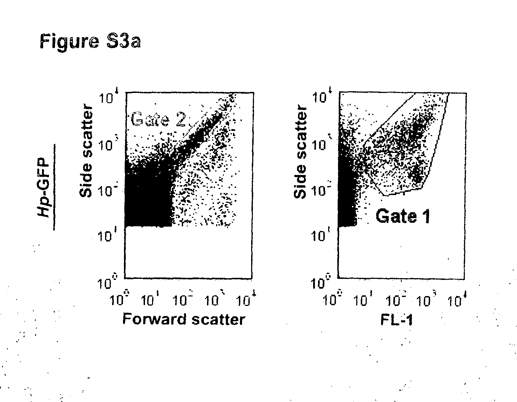

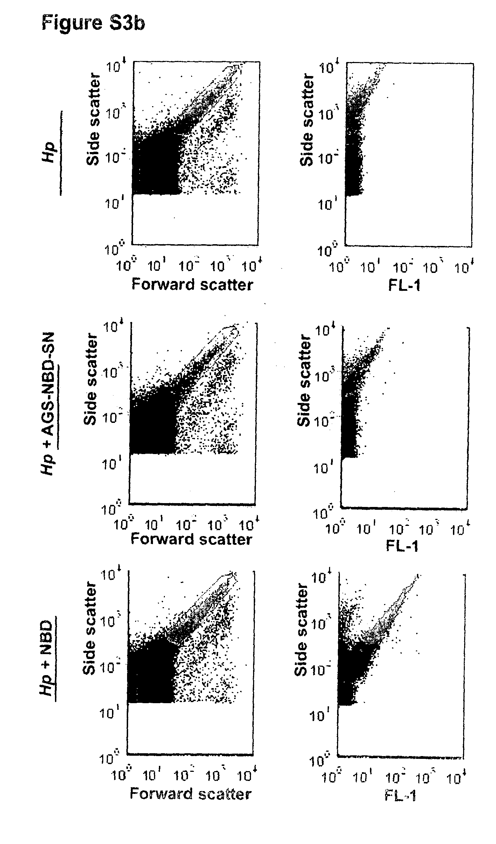

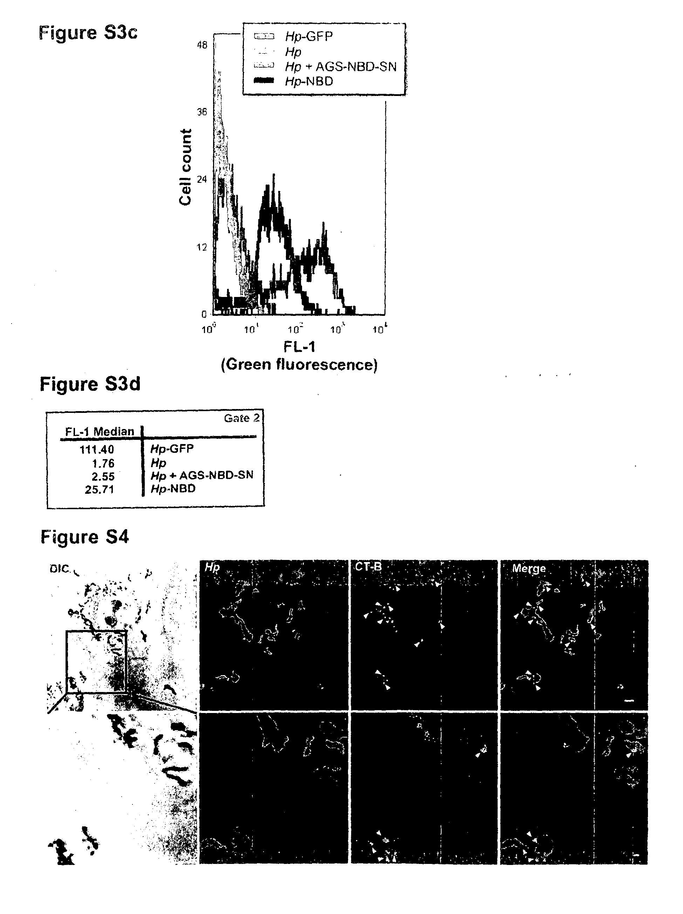

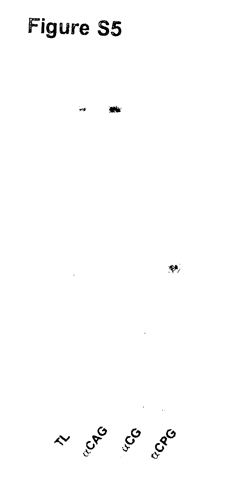

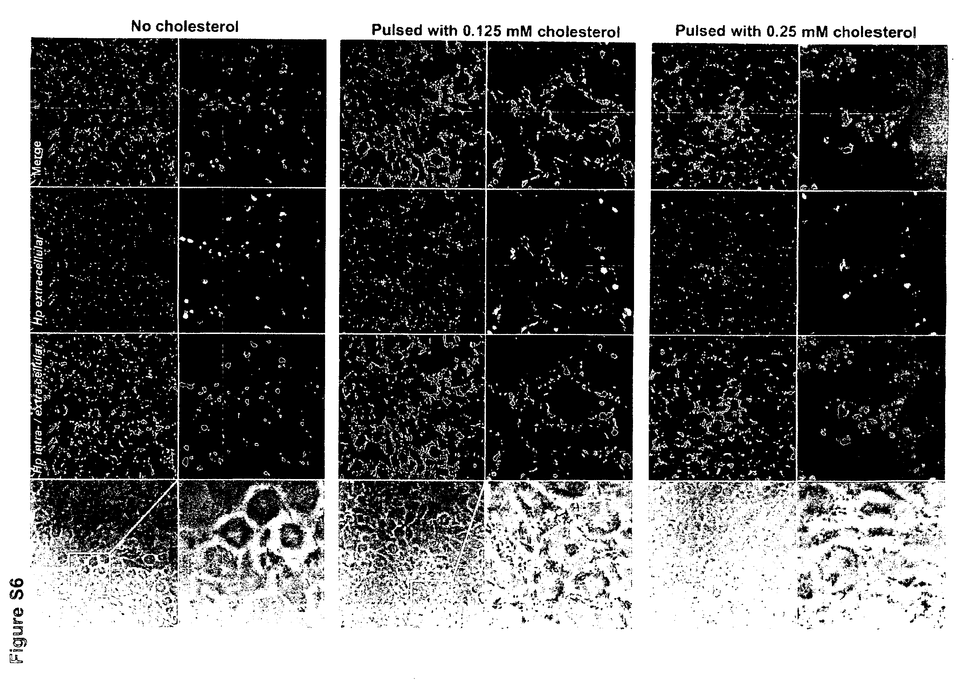

Figure 5a demonstrates complete absence of cholesterol glucosides from Δ421 mutants compared to wild-type *H. pylori* producing the three forms of glucosides. Reconstitution of Δ421 mutants with the gene coding for cholesteryl-α-glucosyltransferase fully restored synthesis of cholesterol glucosides (Fig. 5a).

To demonstrate the function of cholesteryl-α-glucosyltransferase in immune evasion and on phagocytosis of *H. pylori,* we infected macrophages with wild type, Δ421 or the reconstitutant *H. pylori.* Using a gentamicin protection assay (Fig. 5b) or differential immunofluorescence staining (Fig. 5c), the amount of adherent or phagocytosed bacteria was determined by CFU counting. Engulfment of bacteria by macrophages was vigorously enhanced in the absence of cholesteryl-α-glucosyltransferase (Δ421) compared to wild-type *H. pylori* as indicated by intracellular bacterial counts (Fig. 5 b, c). Reconstitution of mutant bacteria with cholesteryl-α-glucosyltransferase fully abolished increased bacterial uptake by macrophages comparable with the phagocytosis rate of wild-type *H. pylori* (Fig. 5a, b). Thus, cholesteryl-α-glucosyltransferase is critically required for escape of *H. pylori* from phagocytosis.

Consistent with our previous observations, cholesteryl-α-glucosyltransferase causes immune evasion in vitro and in vivo. To address the effect of infection with *H. pylori* lacking cholesteryl-α-glucosyltransferase on antigen-specific T cell activation, APCs were infected with wild type, Δ421 mutant or reconstitutant *H. pylori* before coculture with T cells from spleen of C57BU6 mice immunized with *H. pylori* protein extract. Two days after stimulation, cells were pulsed with 3H-thymidine for subsequent analysis of T cell proliferation (Fig. 5c). Non-infected APCs were pulsed with *H. pylori* protein and induced activation of T cells from immunized mice in contrast to naïve animals indicating antigen-specific T cell responses (Fig. 5c). Infection of APCs using wild-type *H. pylori* elicited weak T cell activation in an MHC-restricted fashion as demonstrated by addition of blocking antibodies against MHC-I and MHC-II compared to isotype controls (Fig. 5c). In marked contrast, infection of APCs with the Δ421 mutant *H. pylori* induced potent MHC-restricted T cell activation (Fig. 5c). Reconstitution of the Δ421 mutant with cholesteryl-α-glucosyltransferase used for infection reduced the magnitude of T cell activation to the level of stimulation by APCs infected with wild-type *H. pylori.* In conclusion, cholesteryl-α-glucosyltransferase of *H*. *pylori* triggers avoidance of T cell responses.

To examine the impact of cholesteryl-α-glucosyltransferase upon *H. pylori* infection in vivo, we orally infected C57BL/6 mice with wild type, Δ421 mutant or reconstitutant *H. pylori.* Different time points after infection, stomachs of experimental animals were collected to determine the bacterial burden by counting CFU. Figure 5d demonstrates that wild-type *H. pylori* properly colonizes the gastric mucosa 48 h after infection. Strikingly, at this early time point post infection, *H. pylori* mutants lacking cholesteryl-α-glycosyltransferase were already cleared from gastric tissue (Fig. 5d). However, Δ421 mutants reconstituted with cholesteryl-α-glucosyltransferase infected stomach tissue comparable to wild-type *H. pylori.* Thus, α-glucosylation of cholesterol is vital for robust *H. pylori* infection in vivo.

We thus report on the identification, cloning, and functional characterization of the cholesterol-α-glucosyltransferase from *H. pylori* and demonstrate the essential role of this enzyme in gastric *H. pylori* infection. Based on these observations it is conceivable that the HP0421 cholesteryl-α-glucosyltransferase constitutes an ideal target for the treatment of both acute and chronic *Helicobacter pylori* infections. The gene encoding this bacterial enzyme has now been identified, cloned and functionally expressed in several heterologous cells. It has also been purified in active form from such recombinant cells such that it is now amenable for functional studies. Moreover, since we have demonstrated the essential character of this enzyme for the pathogenic properties and the infectivity by *H. pylori,* it is now useful and possible to prepare cellular extracts containing the enzyme or purified enzyme in large quantities. Such preparations may serve as a basis to generate crystals in order to study the structure of the enzyme and to facilitate the design of inhibitory drugs. Alternatively, such preparations are useful to screen for the inhibitory features of any chemically or biologically derived compound. Once, compounds expressing inhibitory features have been identified they can be suitably optimised by any available approach of rational drug design.

Taking into account the above-described experimental results demonstrating that
- H. pylori acquires cholesterol from the plasma membrane of epithelial cells,
- H. pylori associates with cholesterol rich membrane domains,
- cholesterol facilitates phagocytosis of H. pylori and subsequent T cell activation,
- a cholesterol rich diet reduces the gastic colonization by H. pylori,
- glucosylation of cholesterol by cholesteryl-alpha-glucosyltransferase of H. pylori mediates immune evasion, and
- the gene HP0421 encodes for the cholesteryl-alpha-glucosyltransferase of H. pylori,
the cholesteryl-alpha-glucosyltransferase of H. pylori is a target for the prevention or/and treatment of a Helicobacter infection or/and a Helicobacter associated disease.

A first aspect of the present invention is a pharmaceutical composition comprising an inhibitor of a cholesteryl alpha-glucosyltransferase, optionally together with a pharmaceutically acceptable carrier, diluent or/and adjuvant, preferably for the prevention or/and treatment of a Helicobacter infection or/and a Helicobacter associated disease.

Yet another aspect is the use of an inhibitor of a cholesteryl alpha-glucosyltransferase for the manufacture of a medicament for the prevention or/and treatment of a Helicobacter infection or/and a Helicobacter associated disease.

The term "cholesteryl alpha-glucosyltransferase" as used in the present invention means any polypeptide capable of catalysing a transfer of a glucose moiety to the hydroxyl group at the 3-position of cholesterol with an alpha-glycosidic linkage. Preferably, the cholesteryl alpha-glucosyltransferase is an enzyme that employs UDP-glucose and cholesterol as substrates.

Preferably, the cholesteryl alpha-glucosyltransferase of the present invention is a Helicobacter cholesteryl alpha-glucosyltransferase, a fragment or/and homologue thereof, more preferably the Helicobacter cholesteryl alpha-glucosyltransferase HP0421, the hypothetical Helicobacter cholesteryl alpha-glucosyltransferase jhp0963, the hypothetical *Helicobacter* cholesteryl alpha-glucosyl transferase HH0676, a fragment or/and homologue thereof.

The fragments and homolgues of the cholesteryl alpha-glucosyltransferase of the present invention may exhibit cholesteryl alpha-glucosyltransferase activity. Based upon the recombinant expression of HP0421 in E. coli and the test of cholesteryl alpha-glucosyltransferase activity of the recombinanty expressed protein as described herein, the person skilled in the art can easily determine if a fragment or homologue of a cholesteryl alpha-glucosyltransferase exhibits cholesteryl alpha-glucosyltransferase activity.

The sequence of the HP0421 cholesteryl alpha-glucosyltransferase is described in SEQ ID NO:2.

The sequence of the hypothetical cholesteryl alpha-glucosyltransferase jhp0963 of *H.pylori* strain J99 is described in SEQ ID NO:4.

The sequence of the hypothetical cholesteryl alpha-glucosyltransferase HH0676 of *Helicobacter hepaticus* ATCC 51449 is described in SEQ ID NO:6.

Homologues of the cholesteryl alpha-glucosyltransferase of the present invention, in particular of SEQ ID NO:2, 4 or/and 6, may have at least 80%, preferably at least 90 %, more preferably at least 95 %, most preferably at least 99 % identical amino acid positions with reference to the length of the sequence of maximal overlap with the respective cholesterol alpha-glucosyltransferase.

Homologues or fragments of the cholesteryl alpha-glucosyltransferase of the present invention, in particular of SEQ ID NO:2, 4 or/and 6, may have a length of at least 50 amino acids, at least 100 amino acids, at least 200 amino acids, at least 300 amino acids or may have the full length of the respective cholesterol alpha-glucosyltransferase.

Homologues or fragments of the cholesteryl alpha-glucosyltransferase of the present invention, in particular of SEQ ID NO:2, 4 or/and 6, may have a length of at the maximum the full length of the respective cholesterol alpha-glucosyltransferase, at the maximum 300 amino acids, at the maximum 200 amino acids, or at the maximum 100 amino acids.

For determination of the number of identical positions of nucleic acid sequences (see below) or amino acid sequences, the sequence of maximal overlap can be determined by a person skilled in the art, e.g. by commonly known algorithms, such as BLAST, BLASTP or algorithms derived thereof.

The inhibitor of the cholesteryl alpha-glucosyltransferase may be any compound which is capable of decreasing the activity of a cholesteryl alpha-glucosyltransferase.

"Activity of a cholesteryl alpha-glucosyltransferase" as used herein refers to the catalysis of a transfer of a glucose moiety to the hydroxyl group at the 3-position of cholesterol with an alpha-glycosidic linkage in order to form cholesterol-α-glucoside. Also included is the biosynthesis of the glycolipids cholesterol-6-O-acyl-α-glucoside, cholesterol-6-O-phosphatidyl-α-glucoside, and cholesterol-6-O-lysophosphatidyl-α-glucoside.

The cholesteryl alpha-glucosyltransferase inhibition may be a specific inhibition. "Specific inhibition" is a selective inhibition of the cholesteryl alpha-glucosyltransferase activity. In a specific inhibition of cholesteryl alpha-glucosyltransferase, the activity of other cellular components is not significantly inhibited, i.e. the IC50 values of the specific cholesteryl alpha-glucosyltransferase inhibitor to other cellular components are at least a factor of 10, preferably a factor of 100, more preferably a factor of 1000 larger than the IC50 values to cholesteryl alpha-glucosyltransferase. The present invention also refers to non-specific inhibition of cholesteryl alpha-glucosyltransferase.

In the present invention, the activity of cholesteryl alpha-glucosyltransferase of the present invention may be inhibited on the nucleic acid level, e.g. on the gene level or on the transcription level. Inhibition on the gene level may comprise a partial or complete gene inactivation, e.g. by gene disruption. Inhibition may also occur on the transcript level, e.g. by application of anti-sense molecules, ribozymes, siRNA molecules. Suitable anti-sense molecules may be selected from DNA molecules, RNA molecules or nucleic acid analogues. Ribozymes may be selected from RNA molecules and nucleic acid analogues. Small RNA molecules capable of RNA interference (siRNA molecules) may be selected from RNA molecules and nucleic acid analogues. The person skilled in the art knows methods for construction of suitable anti-sense molecules or/and siRNA molecules from the respective cholesteryl alpha-glucosyltransferase sequences.

SEQ ID NO:1 describes a nucleotide sequence encoding the HP0421 cholesteryl alpha-glucosyltransferase. Based upon SEQ ID NO:1 and the amino acid sequence SEQ ID NO:2, a person skilled in the art is able to construct siRNA molecules capable of inhibiting the HP0421 cholesteryl alpha-glucosyltransferase activity.

SEQ ID NO:3 describes a nucleotide sequence encoding the hypothetical jhp0963 cholesteryl alpha-glucosyltransferase of *H. pylori* strain J99. Based upon SEQ ID NO:3 and the amino acid sequence SEQ ID NO:4, a person skilled in the art is able to construct siRNA molecules capable of inhibiting the hypothetical jhp0963 cholesteryl alpha-glucosyltransferase activity.

SEQ ID NO:5 describes a nucleotide sequence encoding the hypothetical HH0676 cholesteryl alpha-glucosyltransferase of *Helicobacter hepaticus* ATCC51449. Based upon SEQ ID NO:5 and the amino acid sequence SEQ ID NO:6, a person skilled in the art is able to construct siRNA molecules capable of inhibiting the HH0676 cholesteryl alpha-glucosyltransferase activity.

The cholesteryl alpha-glucosyltransferase activity may also be inhibited on the protein level, e.g. by application of compounds which result in a specific or unspecific cholesteryl alpha-glucosyltransferase inhibition.

The inhibition may comprise, for example, the application of antibodies or antibody fragments directed against cholesteryl alpha-glucosyltransferase. The antibodies may be polyclonal antibodies or monoclonal antibodies, recombinant antibodies, e.g. single chain antibodies or fragments of such antibodies which contain at least one antigen-binding site, e.g. proteolytic antibody fragments such as Fab, Fab' or F(ab')₂ fragments or recombinant antibody fragments such as scFv fragments. For therapeutic purposes, particularly for the treatment of humans, the application of chimeric antibodies, humanized antibodies or human antibodies is especially preferred.

Furthermore, proteinaceous or low-molecular weight cholesteryl alpha-glucosyltransferase inhibitors may be used in the present invention.

Cholesteryl alpha-glucosyltransferase inhibitor as used herein also includes a combination of at least two cholesteryl alpha-glucosyltransferase inhibitors as defined above, in particular two, three, four, or five cholesteryl alpha-glucosyltransferase inhibitors.

Further cholesteryl alpha-glucosyltransferase inhibitors may be identified by a screening method of the present invention as outlined in detail below.

Kawakubo et al. (Science 305, 1003-6, 2004) describe that CD43 containing a terminal α1,4-N-acetyl glucosamine and p-nitrophenyl-alpha-N-acetylglucosamine (GlcNAc-alpha-PNP or PNP-GlcNAc) suppressed the growth of *H. pylori.* The authors describe abnormal alterations of cell morphology upon treatment with CD43 containing a terminal α1,4N-acetyl glucosamine and suggest inhibition of cell wall biosynthesis as a possible mechanism.

Kawakubo et al. (supra) further describe that soluble CD43 containing a terminal α1,4-N-acetylglucosamine inhibits the formation of cholesteryl-alpha-glucoside in a Helicobacter cell lysate. The authors conclude that this inhibitory effect is due to the inhibition of the α-glucosyltransferase contained in the Helicobacter lysate by an end-product inhibition mechanism.

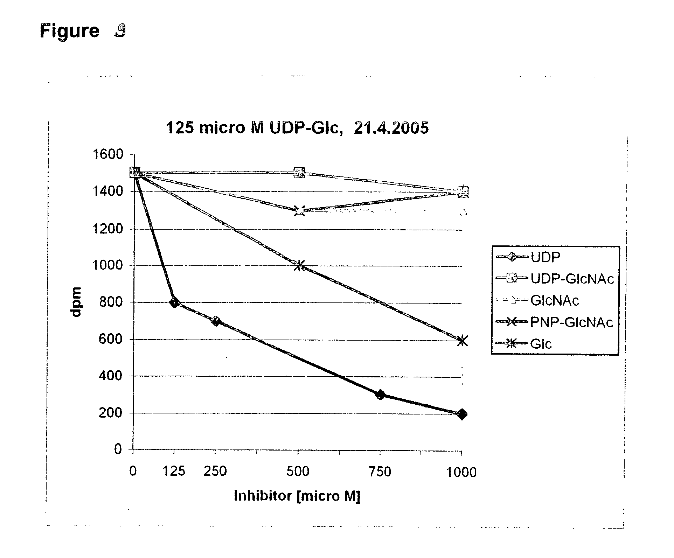

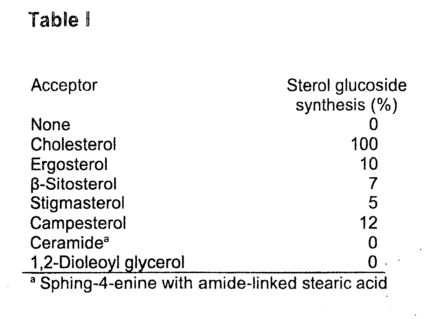

The inventors of the present invention performed experiments demonstrating that PNP-GlcNAc, UDP-GlcNAc and GlcNAc in a concentration up to 1 mM do not inhibit recombinant cholesteryl alpha glucosyltransferase HP0421, which is the only cholesteryl alpha glucosyltransferase of H. pylori (results summarized in Fig. 9). From these results, the inventors of the present invention conclude that the growth inhibition of H. pylori by soluble CD43 carrying a terminal α1,4-N-acetyl glucosamine group and PNP-GIcNAc is probably not due to an inhibition of the H. pylori cholesteryl alpha glucosyltransferase. It is unclear by which mechanism the production of cholesterol glucosides is inhibited in the Helicobacter cell lysate of Kawakubo et al. (supra).

Further, the inventors of the present invention demonstrated that H. pylori HP0421 knock-out mutants (hereinafter termed as Hp Δ421) incapable of expressing a functional cholesteryl alpha glucosyltransferase exhibit an almost unaltered morphology compared with H. pylori wild-type. Further Hp Δ421 is growing in vitro as well as in vivo. If the cholesteryl alpha glucosyltransferase would be responsible for the morphological changes and the growth inhibition observed by Kawakubo et al. (supra), the Hp Δ421 mutant of the present invention should exhibit a similar morphology and growth inhibition. Since this is not the case, it must be concluded that the inhibition of cell wall synthesis and growth observed by Kawakubo et al. (supra) must be mediated by a target different from the cholesteryl alpha glucosyltransferase.

No disclosure can be found in Kawakubo et al. (supra) that cholesterol metabolism of Helicobacter plays an important role in escape from the host immune system in Helicobacter infections or/and Helicobacter associated diseases. There is no disclose that cholesteryl alpha-glucosyltransferase and cholesterol glucosides are essential for escape of Helicobacter from phagocytosis.

The inhibitor of the cholesteryl alpha-glucosyltransferase of the present invention preferably does not comprise an N-acetylglucosamine group such as an alpha-linked N-acetylglucosamine group. More preferably, the inhibitor of the present invention does not comprise a glycan carrying a terminal alpha-1,4-linked N-acetylglucosamine group and does not comprise p-nitrophenyl-alpha-N-acetylglucosamine. Even more preferably, the inhibitor of the present invention does not comprise an O-glycan carrying a terminal alpha-1,4-linked N-acetylglucosamine group and does not comprise p-nitrophenyl-alpha-N-acetylglucosamine. Most preferably, the inhibitor of the present invention does not comprise CD34 comprising an O-glycan carrying a terminal alpha-1,4-linked N-acetylglucosamine group, CD43 comprising an O-glycan carrying a terminal alpha-1,4-linked N-acetylglucosamine group and p-nitrophenyl-alpha-N-acetylglucosamine.

In the context of the present invention the Helicobacter infection to be treated by an inhibitor of the cholesteryl alpha-glucosyltransferase of the present invention is preferably an infection with Helicobacter pylori. A Helicobacter associated disease is preferably selected from chronic gastritis, peptic ulcer disease, and cancer, more particular gastric cancer such as MALT lymphoma or gastric adenocarcinoma.

Yet another aspect of the present invention is a combination of the administration of the inhibitor of the present invention with a further therapy for the prevention or/and treatment of a Helicobacter infection or/and a Helicobacter associated disease. Therefore, the pharmaceutical composition comprising an inhibitor of the present invention or/and the medicament of the present invention for the prevention or/and treatment of a Helicobacter infection or/and Helicobacter associated disease are manufactured for the combination with a further therapy for the prevention or/and treatment of a Helicobacter infection or/and Helicobacter associated disease.

The further therapy may be any known therapy of a Helicobacter infection or/and a Helicobacter-associated disease. Preferably, the further therapy comprises the administration of at least one antibiotic. More preferred is the so-called "triple therapy", which comprises the administration of a combination of two antibiotics, which may be selected from amoxicillin, tetracycline, metronidazole and clarithromycin, and a proton pump inhibitor which may be selected from omeprazole and pantoprazole.

The further therapy may also comprise a stimulation of the immune system, such as an unspecific or a Helicobacter specific stimulation of the immune system. In particular, the further therapy may comprise a vaccination against a Helicobacter infection. Suitable vaccines may comprise the Helicobacter proteins urease, HP0410, HP0231, homologues or/and fragments thereof as antigens. The vaccine may be a recombinant subunit vaccine, a live vaccine, or a nucleic acid vaccine. The live vaccine may be based on an attenuated microbial cell, in particular on an attenuated bacterial cell, more particularly on an attenuated gram negative bacterial cell, most particularly on a Salmonella cell.

A vaccine comprising the Helicobacter polypeptides HP0410 or/and HP0231 is described in WO 01/83531, which is included herein by reference. A recombinant live vaccine comprising the Helicobacter urease (subunits A or/and B) is described in WO 98/16552, which is included herein by reference. The live vaccine described in WO 98/16552 is based upon an attenuated microbial cell, in particular on a Salmonella cell.

Yet another aspect of the present invention is a cholesterol-rich diet. As demonstrated by the experimental results as described above, a cholesterol-rich diet reduces gastric colonization by Helicobacter. Therefore, a cholesterol-rich diet is suitable for the treatment of a Helicobacter infection or/and a Helicobacter associated disease as described above. Cholesterol may therefore be used for the manufacture of a medicament for the treatment of a Helicobacter infection or/and a Helicobacter associated disease.

The cholesterol-rich diet may also be combined with any therapy for treatment of a Helicobacter infection or/and a Helicobacter associated disease, such as the state of the art treatment regimens as described above. The cholesterol rich may in particular be combined with administration of at least one antibiotic as described above, or/and with a stimulation of the immune system, such as an unspecific or a Helicobacter specific stimulation of the immune system, such as a vaccination.

In a preferred embodiment of the present invention, the inhibitor of the cholesteryl alpha-glucosyltransferase may be administered together with a cholesterol rich diet. The inhibitor of the cholesteryl alpha-glucosyltransferase may be used for the manufacture of a medicament for the prevention or/and treatment of a Helicobacter infection or/and a Helicobacter associated disease, which medicament is for administration in combination with a cholesterol rich diet.

The amount of cholesterol to be administered to a subject in need thereof in a cholesterol-rich diet may be adjusted a reach a total content of cholesterol in the food of from about 0.5 % by weight up to about 5 % by weight, preferably of from about 1 % by weight up to about 4 % by weight, more preferably of from about 2 % by weight up to about 3 % by weight, most preferably of from about 2 % by weight up to about 2,5 % by weight. In terms of daily doses, the amount of cholesterol to be administered to a subject in need thereof in a cholesterol-rich diet may be from about 1 to about 10 g/day, preferably from about 2 to about 8 g/day, more preferably from about 4 to about 6 g/day, most preferably from about 4 to about 5 g/day.

Cholesterol may be administered to a subject in need thereof in the form of a cholesterol containing pharmaceutical composition.

The cholesterol-rich diet may be continued for up to 4 weeks, preferably up to 2 weeks, more preferably up to 1 week. The duration of the cholesterol-rich diet may also depend upon the duration of the further therapy as discussed above. The duration of the cholesterol-rich diet may also depend upon the duration of the administration of the cholesteryl alpha-glucosyltransferase inhibitor.

Yet another aspect of the present invention is a pharmaceutical composition comprising a cholesteryl alpha glucosyltransferase, a nucleic acid encoding a cholesteryl alpha glucosyltransferase, a fragment or/and homologue thereof, optionally together with a pharmaceutically acceptable carrier, diluent or/and adjuvant. A cholesteryl alpha glucosyltransferase, a nucleic acid encoding a cholesteryl alpha glucosyltransferase, a fragment or/and a homologue thereof may be used for the manufacture of a medicament for the prevention or/and treatment of a Helicobacter infection or/and a Helicobacter associated disease.

In a preferred embodiment, the pharmaceutical composition comprising a cholesteryl alpha glucosyltransferase, a nucleic acid encoding a cholesteryl alpha glucosyltransferase a fragment or/and a homologue thereof is an immunogenic composition, more preferably a vaccine.

In yet another preferred embodiment, in the pharmaceutical composition, the cholesteryl alpha-glucosyltransferase is a Helicobacter cholesteryl alpha-glucosyltransferase, a fragment or/and a homologue thereof, more preferably Helicobacter cholesteryl alpha-glucosyltransferase HP0421, jhp0963, HH0676, a fragment or/and a homologue thereof.

In a further preferred embodiment, the pharmaceutical composition comprising a cholesteryl alpha glucosyltransferase, a nucleic acid encoding a cholesteryl alpha glucosyltransferase, a fragment or/and a homologue thereof is for the prevention or/and treatment of a Helicobacter infection or/and a Helicobacter associated disease.

In yet another preferred embodiment, the pharmaceutical composition comprising a cholesteryl alpha glucosyltransferase, a nucleic acid encoding a cholesteryl alpha glucosyltransferase, a fragment or/and a homologue thereof is for combination with a further therapy for the prevention or/and treatment of a Helicobacter infection or/and a Helicobacter-associated disease or/and for combination with a cholesterol-rich diet.

The cholesteryl alpha glucosyltransferase, fragments or/and homologues thereof which may be used in the pharmaceutical composition are as described above.

The nucleic acid sequence which may be used in the pharmaceutical composition may be any nucleic acid encoding a cholesteryl alpha glucosyltransferase, a fragment or/and homologue thereof, which cholesteryl alpha glucosyltransferase, a fragment or/and homologue thereof is as described above.

Preferably, the nucleic acid comprises the sequence SEQ ID NO:1, 3 or/and 5, a sequence capable of hybridizing under stringent conditions with SEQ ID NO:1, 3 or/and 5, a sequence within the scope of the degeneracy of the genetic code of SEQ ID NO:1, 3 or/and 5, a fragment or/and a homologue thereof.

The homologue of a nucleic acid encoding a cholesteryl alpha-glucosyltransferase of the present invention, in particular of SEQ ID NO:1, 3 or/and 5, may have at least 80%, preferably at least 90 %, more preferably at least 95 %, most preferably at least 99 % identical nucleotide positions with reference to the length of the sequence of maximal overlap with the nucleic acid encoding the respective cholesterol alpha-glucosyltransferase.

The fragment or homologue of a nucleic acid encoding a cholesteryl alpha-glucosyltransferase, in particular of SEQ ID NO:1, 3 or/and 5, may have a length of at least 10 nucleotides, at least 20 nucleotides, at least 50 nucleotides, at least 100 nucleotides, or at least 200 nucleotides, or may have the full length of the nucleic acid encoding the respective cholesteryl alpha-glucosyltransferase.

The fragment or homologue of a nucleic acid encoding a cholesteryl alpha-glucosyltransferase, in particular of SEQ ID NO:1, 3 or/and 5, may have a length of at the maximum the full length of the nucleic acid encoding the respective cholesteryl alpha-glucosyltransferase, at the maximum 1000 nucleotides, at the maximum 500 nucleotides, or at the maximum 200 nucleotides.

A person skilled in the art knows stringent hybridization conditions (see e. g. Sambrook J. et al., 1989, Molecular Cloning, A Laboratory Manual, Cold Spring Harbor Press, Plainview, N.Y). Hybridization under stringent conditions includes washing for 1 h with 1 x SSC and 0.1 % SDS at 50°C, preferably at 55°C, more preferably at 62°C and most preferably at 68°C, particularly for 1 h in 0.2 x SSC and 0.1% SDS at 50°C, preferably at 55°C, more preferably at 62°C, and most preferably at 68°C, wherein a positive hybridization signal is observed.

Yet another aspect of the present invention is a method for prevention or/and treatment of a Helicobacter infection or/and a Helicobacter associated disease comprising administering an effective amount of a medicament for the prevention or/and treatment of a Helicobacter infection or/and a Helicobacter associated disease as described above or/and the pharmaceutical composition of the present invention to a subject in need thereof.

In a preferred embodiment, the method of the present invention is combined with a further therapy for the prevention or/and treatment of a Helicobacter infection or/and a Helicobacter-associated disease.

In yet another preferred embodiment, the method of the present invention is combined with a cholesterol-rich diet.

A further aspect of the present invention is a method for enhancing an immune response against a Helicobacter infection comprising administering a cholesterol-rich diet to a subject in need thereof.

In a preferred embodiment, the method for enhancing an immune response increases phagocytosis of Helicobacter cells by antigen presenting cells or/and increases T cell activation. Preferably, phagocytosis is phagocytosis by antigen presenting cells such as macrophages and dendritic cells.

In a preferred embodiment, the method for enhancing an immune response comprises inhibiting cholesterol degradation or/and conversion into cholesterol glucosides in a *Helicobacter* cell.

Yet another aspect of the present invention is the use of a cholesteryl alpha-glucosyltransferase, a nucleic acid encoding a cholesteryl alpha-glucosyltransferase, a fragment or/and a homologue thereof for the identification of an agent for the prevention or/and treatment of a Helicobacter infection or/and a Helicobacter associated disease.

A further aspect of the present invention is a screening method for the identification of an agent for the prevention or/and treatment of a Helicobacter infection or/and a Helicobacter associated disease, comprising identifying a cholesteryl alpha-glucosyltransferase inhibitor.

In a particular embodiment, in the screening method of the present invention, identifying the cholesteryl alpha-glucosyltransferase inhibitor comprises the steps
(a) contacting at least two compounds with a cholesteryl alpha-glucosyltransferase or/and with a nucleic acid encoding a cholesteryl alpha-glucosyltransferase,
(b) determining the cholesteryl alpha-glucosyltransferase activity, and
(c) selecting a compound of (a) capable of inhibiting the cholesteryl alpha-glucosyltransferase activity in (b).

In the screening method of the present invention, the cholesteryl alpha-glucosyltransferase may be any cholesteryl alpha-glucosyltransferase as described above, including fragments and homologues thereof which may have cholesteryl alpha-glucosyltransferase activity. Preferably, in the screening method, the cholesteryl alpha-glucosyltransferase is a *Helicobacter* cholesteryl alpha-glucosyltransferase, more preferably *Helicobacter* cholesteryl alpha-glucosyltransferase HP0421, jhp0963, HH0676, a fragment or/and a homologue thereof.

In the screening method of the present invention, a nucleotide sequence may be employed encoding a cholesteryl alpha-glucosyltransferase of the present invention, in particular a nucleotide sequence may be employed encoding the cholesteryl alpha-glucosyltransferase as described by SEQ ID NO: 1, 3 or/and 5, a sequence capable of hybridizing under stringent conditions with SEQ ID NO:1, 3 or/and 5, a sequence within the scope of the degeneracy of the genetic code of SEQ ID NO:1, 3 or/and 5, a fragment or/and a homologue thereof.

The screening method of the present invention, e.g. step (a) or/and (b) of the particular embodiment as described above, may comprise the use of isolated proteins, cell extracts, recombinant cells or/and transgenic non-human animals. In particular, the cholesteryl alpha-glucosyltransferase inhibitor may be identified in a molecular or/and cellular assay.

The recombinant cells or/and transgenic non-human animal preferably exhibit an altered cholesteryl alpha-glucosyltransferase expression, in particular an cholesteryl alpha-glucosyltransferase overexpression compared to a corresponding wild-type cell or/and animal.

In the screening method of the present invention, the cholesteryl alpha-glucosyltransferase inhibitor is an inhibitor as discussed above, i.e. an inhibitor capable of decreasing the activity of a cholesteryl alpha-glucosyltransferase. Preferably, the inhibitor inhibits catalysing a transfer of a glucose moiety to the hydroxyl group at the 3-position of cholesterol with an alpha-glycosidic linkage. In an alternative preferred embodiment, the inhibitor reduces the amount of cholesteryl alpha-glucosyltransferase. Thus, the screening method, e.g. step (b) of the particular embodiment described above, comprises determination of transfer of a glucose moiety to the hydroxyl group at the 3-position of cholesterol with an alpha-glycosidic linkage or/and the determination of the amount of cholesteryl alpha-glucosyltransferase.

In the screening method, inhibition of a cholesteryl alpha-glucosyltransferase may be determined by inhibition of the biosynthesis of cholesterol-α-glucoside, cholesterol-6-O-acyl-α-glucoside, cholesterol-6-O-phosphatidyl-α-glucoside, or/and cholesterol-6-O-lysophosphatidyl-α-glucoside.

In the screening method of the present invention, any reactant required for the biosynthesis of cholesterol-α-glucoside, cholesterol-6-O-acyl-α-glucoside, cholesterol-6-O-phosphatidyl-α-glucoside, or/and cholesterol-6-O-lysophosphatidyl-α-glucoside may be employed, such as UDP glucose or/and cholesterol. Also employed may be a derivative of a reactant, such as a derivative resulting in a product which does not naturally occur and which therefore can be easily detected. Preferred is a labelled reactant, such as a reactant carrying a luminescent label which can be easily determined by photometric methods.

The screening method of the present invention is suitable as a screening procedure, e.g. a high-through put screening procedure for identifying novel compounds or classes of compounds. Further, the screening method of the present invention is suitable as a validation procedure for characterizing the pharmaceutical efficacy and/or the side effects of compounds.

### The methods employed in the experiments described above are as follows:

### Methods

All reagents were purchased from Sigma, unless otherwise indicated.

**Bacterial strains and cholesterol loading**. *H. pylori (Hp)* strains P12, P12Δ*cag*PAI, P12-GFP, P12Δ*FlgE* and the mouse-adapted strain Hp76, Hp76Δ*421* and Hp76Δ*421*reconst. were grown as described before (Gomez-Duarte, O.G. et al., Vaccine 16, 460-471, 1998). *Hp* P12 was transformed with pHel3 to become P12-GFP. The non-motile mutant P12Δ*FlgE* strain was constructed by replacement of *flgE* gene (GeneID: 890091) with kanamycin resistance cassette as described before (Heuermann, D. & Haas, R., Mol. Gen. Genet. 257, 519-528, 1998). The cholesteryl-α-glucosyltransferase negativ mutant Hp76Δ*421* was constructed by replacement of *hp0421* gene (GeneID: 900074) with chloramphenicol resistance cassette. Hp76Δ*421* was transformed with pHel3-SGT (kanamycin resistance) to become Hp76Δ*421*reconst. *Salmonella typhimurium, E. coli* (DH5α) or *Shigella flexneri* were grown aerobically at 37°C in Luria-Bertani medium. For cholesterol loading, bacteria were pulsed with water-soluble cholesterol, purified cholesteryl-α-glucoside from Hp or cholesteryl-β-glucoside (Research Plus, Manasquan, USA) [solubilized in (2-hydroxypropyl)-β-cyclodextrin] for 30 min and washed twice with RPMI. Bacteria were either immediately used for infection and TLC analysis or were grown for indicated time points in RPMI under microaerophilic conditions.

Cell culture and infection. AGS cells (ATCC, CRL-1739) and the mouse macrophage cell line J774A.1 (ATCC, TIB-67) were maintained in RPMI medium 1640 (Gibco) with 10% FCS. Mouse macrophages were generated from bone marrow cells of C57BL/6 mice (Schaible, U.E. et al., J. Cell. Sci. 112 (Pt5), 681-693, 1999). Transfection was performed with Lipofectamine 2000 (Invitrogen). Cells were deprived of serum for 12 h before infection. If indicated, AGS cells were treated with 5 mg/mL of methyl-β-cyclodextrin in RPMI for 30 min for cholesterol depletion and washed twice with RPMI. Depletion revealed a cholesterol lowering effect of ~36%. Human AGS cells were infected with Hp P12 or P12 mutant strains, whereas mouse macrophages were infected with *Hp76.* Infection was performed at an MOI of 100 (for microscopical analysis and T cell proliferation an MOI of 30). For synchronized infection, bacteria were centrifuged onto the cells for 5 min at 800 g at room temperature.

**Chemotaxis assay**. A hermetic tube system was constructed with three tubes containing test substance, *Hp* in RPMI (3 x 10⁸/mL) or RPMI, respectively (Fig. S1). For the chemotaxis assay water-soluble cholesterol, water-soluble 17β-estradiol or L-α-phosphatidylcholine were used as test substances (250 µM). Tube suspensions were collected at different time points, diluted and plated on agar medium for subsequent counting of colony forming units (CFU).

**Isolation of cholesterol-rich membranes.** Cholesterol-rich membranes were isolated as described previously using either detergent-free buffer (Song, K.S. et al., J. Biol. Chem. 271, 9690-9697, 1996) for *Hp* association with membrane domains or detergent extraction (Smart, E.J. et al., J. Cell. Biol. 127, 1185-1197, 1994) for membrane localization of GPI-GFP. Briefly, AGS cells were lysed with 20 strokes using a Dounce homogenizer in 500 mM sodium carbonate (pH 11). The lysates were adjusted to 45% sucrose and overlaid with 6 mL of 35% and 2 mL of 5% sucrose and centrifuged for 18 h at 37,000 rpm 4°C in a SW40Ti rotor (Beckman Instruments, USA). Thirteen 1 mL fractions were collected from the top of the gradient and assayed for flotillin-1 and cholesterol content. Secondly, AGS cells were solubilized in 1% Triton X-100/MBS [Mes-buffered saline; 0.25 M NaCl and 25 mM Mes (pH 6.8)] on ice for 1 h. The lysates were adjusted to 45% sucrose in MBS, overlaid with 2.5 mL of 35% and 0.5 mL of 5% sucrose in MBS, and centrifuged for 2.5 h at 55,000 rpm 4°C in a SW60 rotor (Beckman Instruments, USA). Eight 0.5 mL fractions were collected from the top of the gradient and were assayed for flotillin-1 and GPI-GFP content. GFP (*Hp*-GFP and GPI-GFP) were detected with Fluoroscan Ascent (Thermo Electron Corp., Dreieich, Germany). Sucrose density of each fraction was determined by refractometry.

**Lipid extraction, purification and analysis**. Lipids were extracted with chloroform/methanol (1:2 and 2:1 v/v), separated by TLC on silica gel 60 plates (Merck, Darmstadt, Germany) with chloroform/methanol/ammonia (40:10:4, v/v) or chloroform/methanol/water (70:30:5 v/v) and visualized with Bial's reagent at 110 °C or 13 mM α-naphthol in sulfuric acid/methanol (3/97, v/v) at 160°C. For structural analysis by MS and NMR spectroscopy, glucolipids were purified and peracetylated (Warnecke, D. et al., J. Biol. Chem., 274, 13048-13059, 1999). Electrospray ionization fourier transform ion cyclotron resonance mass spectrometry (ESI FT-ICR MS) and one- (1 D) and two- (2D) dimensional homonuclear [¹H]-NMR spectra and heteronuclear [¹H], [¹³C] , and [³¹P] NMR spectroscopy was performed using a Fourier transform ion cyclotron resonance mass analyzer (Apexll, Bruker Daltonics, USA) equipped with a 7 T actively shielded magnet and an Apollo electrospray ion source as described previously (Holzl, G. et al., Glycobiology 15, 874-886, 2005). Cholesterol concentration was determined with Cholesterol Total-LSR Set (Thermotrace, Australia).

**Construction of GPI-GFP.** The signal peptide of huCD55, aa 1-34 and aa 343-381, which contains the GPI attachment site at aa 353, was ligated upstream and downstream of GFP, respectively. The construct was subsequently cloned into the mammalian expression vector pEGFP-N1 (Clontech). The expressed fusion protein truncated CD55-GFP links to glycosylphosphatidylinositol (GPI) anchor *in vivo* resulting in GPI-GFP.

**Immunoblotting.** Proteins from lysed cells or from trichloroacetic acid precipitates were solubilized in Laemmli buffer, separated by SDS-PAGE, transferred to PVDF membranes, probed with primary [GFP(FL, Santa Cruz Biotechnology) and flotillin-1 (Clon18, BD Biosciences Pharmingen)] and horseradish peroxidase-conjugated secondary antibodies (Amersham) and detected with ECL reagent (ICN).

**Microscopy.** AGS cells or J774A.1 cells were grown on glass coverslips. AGS cells were preloaded with 5 µg/mL of NBD-cholesterol [25-{N-[(7-nitrobenz-2-oxa-1,3-diazol-4-yl)-methyl]amino}-27-norcholesterol, (Avanti Polar Lipids, Alabaster, USA)] for 3 h and washed twice with 5 mg/mL of MβCD and once with RPMI. Cells were infected at an MOI of 30 for 30, 90 or 180 min, fixed in 3.7% paraformaldehyde and blocked with 1% goat serum for immunofluorescence staining. Extracellular bacteria were detected by incubation of the unpermeabilized cells with anti-*Hp* antibodies (USB, Swampscott, USA) followed by secondary antibodies conjugated to Cy-5 dye (Jackson Immunoresearch, West Grove, USA). Intracellular and extracellular bacteria were subsequently stained by permeabilisation using 0.1% Triton-X 100 in 1% goat serum, followed by staining with anti-*Hp* antibodies and secondary antibodies conjugated with Cy-2. GM1 staining was performed with Cholera toxin subunit B-FITC (8 µg/mL) for 1 h, while Hp staining was done with anti-*Hp* antibodies followed by secondary antibodies conjugated to Cy-3. Samples were analyzed by confocal laser scanning microscopy using a Leica TCS SP microscope, equipped with an argon/krypton mixed gas laser source (Leica, Wetzlar, Germany). AGS cells were grown in glass-bottom culture dishes (MatTek, Ashland, USA) for TIRF microscopy. Images were captured using a 60x 1.45-NA Plan Apochromat TIRF-objective and the IX-70 microscope (Olympus, Hamburg, Germany). An argon/krypton laser was used as the light source for excitation.

**Gentamicin protection assay.** To investigate the survival of Hp in macrophages, J774A.1 cells were seeded in 24-well plates infected with bacteria at an MOI of 100. Infection was synchronized by centrifugation for 5 min at 800 g. Cells were incubated for 90 min at 37°C. Extracellular bacteria were killed by addition of 200 µg/mL of gentamicin for 2 h. After washing with PBS, cells were lysed for 5 min with 0.1% saponin in PBS. Total adhering bacteria were determined with saponin lysis without using gentamicin. Appropriate dilutions of the supernatant were plated on agar plates to count colony-forming units (CFU) (Odenbreit, S. et al., Cell. Microbiol. 3, 21-31, 2001).

**T cell assays.** *Hp* lysates were prepared for immunization by using a French press and purified by ultracentrifugation (100,000 g, 1 h). C57BU6 mice were s.c. immunized with 70 µg of Hp lysates with Freund's adjuvant and boosted twice with 35 µg every 2 weeks. T cells were isolated from spleen and lymph nodes 5 days after the last boost. APCs were infected with Hp (pulsed with cholesterol for 30 min or left untreated), incubated with concanavalin A (1 µg/mL) or 10 µg/mL of *Hp* protein (with or without 1 mM cholesterol) for 90 min, treated with 200 µg/mL gentamicin for 2 h and irradiated with 30 Gy before the addition of T cells (1 x 10⁵ cells/well). For MHC blocking experiments 10 µg/mL of MHC-I (clone 28/14/8F) and/or 10 µg/mL MHC-II (clone TIB120) and isotype control (20 µg/mL) antibody was used. Cells were cultured in triplicates in complete medium in 96-well roundbottomed plates for 3 d at 37°C and 5% CO₂. T cells were pulsed with [³H] thymidine (0.5 µCi/well; Amersham, USA) on day 3 and were collected 18 h later for liquid scintillation measurement in a Top Count scintillation counter (Packard).

**Mouse infection model.** Female C57BU6 mice or C57BU6 rag-1 (-/-) (RAG) mice (6 weeks old, Charles River, Sulzfeld, Germany) were treated with a 3,500 kcal/kg balanced 2% or 0% cholesterol diet (custom product, Altromin, Lage, Germany), initiated 14 days before infection. Mice were infected with a single oral dose of 9.5 x 10⁸ Hp76 cells suspended in PBS and 0.1 M NaHC0₃. Control mice were sham-infected. 6 mice of each group were sacrificed at time points 24 h, 2 and/or 10 and 25 weeks post infection. The stomach was removed, and one half of the tissue was weighed, homogenized, serially diluted and plated out on GC agar plates containing vancomycin and streptomycin to determine the number of colony-forming units (CFU) of Hp. The remaining tissue was fixed in 4% paraformaldehyde in PBS for histological analysis or was immediately snap frozen in liquid nitrogen for RNA extraction. All protocols involving animal experiments were approved by the Animal Care and Ethics Committee.

**Histology.** Gastric formalin-fixed tissues were embedded in paraffin, sectioned (thickness 4 µm), and stained with hematoxylin and eosin and the Warthin-Starry technique for assessment of histopathology in accordance with the updated Sydney system (Dixon, M.F. et al., Helicobacter 2 Suppl 1, S17-S24, 1997) in a blinded fashion.

**Microarrays**. RNA extraction, labeling, hybridization and analysis for microarrays was performed as described previously (Walduck, A. et al., FASEB J. 18, 1955-1957, 2004). Briefly, for RNA extraction, the stomach mucosa was scraped off with a sterile scalpel blade on liquid nitrogen until ~2/3 of the tissue was removed. Total RNA was prepared using the RNeasy protocol with on-column DNAse digestion (Qiagen, Hilden, Germany) and confirmed to be intact and free of contaminating DNA using an Agilent 2100 bioanalyzer and a RNA 6000 Nano LabChip kit (Agilent Technologies, Palo Alto, CA). A custom tailored 'in situ' synthesized 60-mer oligonucleotide microarray (Agilent) comprising 8014 murine genes with immunological focus was used for expression profiling. RNA was amplified and labeled using a reverse-transcriptase/T7 polymerase linear amplification labeling kit (Agilent), and 1.5 µg of respective cRNAs labeled either with Cy3 or Cy5 were hybridized for 16 h at 65°C. Slides were used and processed according to the manufacturer's manuals. Features were extracted with Image Analysis software (Version A.7.5.1, Agilent). Intensities were normalized by local background correction and by rank consistency filtering with a combination of linear and LOWESS normalization. Data analysis was performed with Rosetta Resolver Biosoftware (Build 5.1.0.0.163, Rosetta Biosoftware, Seattle, WA). The data discussed in this publication have been deposited in NCBIs Gene Expression Omnibus (GEO, http://www.ncbi.nlm.nih.gov/geo/) and are accessible through GEO Series accession number GSE3878 (Barrett, T. et al., Nucleic Acid Res. 33, D562-D566, 2005).

**Flow cytometry.** To measure bacterial NBD-cholesterol uptake, *Hp* (P12) was incubated with supernatant from NBD-cholesterol loaded AGS cells or RPMI-NBD-cholesterol (1 µg/mL) as positive control for 3 h or 30 min, respectively. Samples were washed in PBS, were fixed in 3.7% PFA and analyzed for green fluorescence (channel FL-1 [515 to 545 nm]) without compensation using a FACScan flow cytometer (Becton Dickinson). Hp cells expressing GFP (P12-GFP) were used for identification and gating of Hp population.

**Bacterial and Yeast Strains, Growth and Recombinant DNA Techniques.** *E*. *coli* strains XL1-Blue (MRF') (Stratagene) and C41(DE3) (Miroux, 1996) were routinely grown aerobically at 37 °C in Luria-Bertani medium (Sambrook 1989). Ampicillin (100 mg·L⁻¹), kanamycin (30 mg·L⁻¹) were included for growth of plasmid-bearing cells. The yeast strain used in this study was *P. pastoris* JC 308 *Δgcs*/*Δugt51B1* (Hillig et al., 2003), grown at 30 °C in YPG medium (10 g·L⁻¹ yeast extract, 20 g·L⁻¹ peptone, 10 ml·L⁻¹ glycerol). For gene expression driven by the AOX1 promoter, 0.5 % methanol was added to minimal medium (13.4 g·L⁻¹ Yeast Nitrogen Base). The vectors pET24d(+) (Novagen), and pPlC3.5 (Invitrogen) were used for cloning. Standard methods were followed for DNA isolation, restriction endonuclease analysis, and ligation (Sambrook et al., 1989).

**Cloning of the Cholesterol-α-Glucosyltransferase from H. Pylori. The** open reading frame (ORF) sequence of the cholesterol-α-glucosyltransferase gene HP0421 **from *H. pylori* 26695 was** amplified from genomic DNA by PCR using the specific oligonucleotide primer pairs HP0421_F (5'- GTACGGATCCACCATGGTTATTGTTTTAGTCGTGGATAG-3' (SEQ ID NO: 7) and HP0421_R (5'-ACGTGGATCCCGTGCGGCCGCTGATAAGGTTTTAAAGAGATGGGGG -3' (SEQ ID NO: 8). Herculase enhanced DNA-polymerase (Stratagene) was used for the amplification of the 1169 bp product containing the entire HP0421 ORF sequence. This amplicon was digested with Neol/BamHl and inserted into pET24d(Ncol/BamHl) leading to pET24d-HP0421 which was used for transformation of *E. coli* C41(DE3) cells. The amplicon was also digested with BamHI/Notl to be inserted into pPIC3.5(BamHI/Notl) resulting in pPIC3.5-HP0421 which was used to transform *P. pastoris.* Exact in-frame cloning and identity of the PCR-cloned fragments were confirmed by sequencing.

**Heterologous Expression of the ORF HP0421 in E. coli and P. pastoris.** *E. coli* C41 (DE3) (Miroux 1996) was used as expression host for the plasmid pET24d-HP0421. After transformation, 50 ml cultures of *E. coli* were grown at 37 °C to an OD₆₀₀ = 0.8-1.2. Induction was performed by adding 0.4 mM isopropyl β-D-thiogalactoside and further incubation for 3 h at 30°C. A P. *pastoris* strain deficient in glycolipid biosynthesis was used to express HP0421: JC 308 *Δgcs::Sh ble, Δugt51B1::URA3, ade1, arg4, his4* (Hillig 2003). In a first step, this strain was transformed with the empty vectors pBLADE and pBLARG (Lin Cereghino 2001) to increase its rate of growth. Subsequently, the resulting strain, DKO (JC 308 *Δgcs::Sh ble, Δugt51B1::URA3, his4)* was used for transformation with pPIC3.5-HP0421 leading to DKO-Hp. Transformed cells were grown at 30°C in 50 ml YPG medium to an OD₆₀₀ between 1 and 2. Expression was driven by the strong AOX1 promoter and was induced by transfering the cells to minimal medium with 0.5 % methanol as sole carbon source followed by additional incubation for 20 h.

*E. coli* or *P. pastoris* cells were harvested by centrifugation (4°C, 10 min, 3200 g), resuspended in 2-3 ml buffer (50 mM Tris/HCI pH 7.5, 5 mM 1,4-dithiothreitol), and cooled in an ice bath. While disruption of *E*. *coli* cells was performed by ultrasonication (probe tip, 10 times for 10 s), 5-8 g of glass beads (Ø 0.4 mm) were added to the yeast cells which were disrupted by vortexing 16 times for 30 s and subsequent ultrasonication (6 times for 10 s). Cell debris were removed by centrifugation (4°C, 10 min, 3200 g) and the supernatant fractions representing the cell-free homogenates were used for *in vitro* assays.

**In Vitro Sterol Glucosyltransferase Assay.** The assay for the cholesterol-α-glucosyltransferase activity was performed similar to the assay for the eukaryotic sterol-α-glucosyltransferases (Warnecke 1994, Warnecke 1999). Shortly, the assay mixture contained in a total volume of 100 µl: 5-30 µl (40-240 µg of protein) of *E*. *coli* or *P. pastoris* cell homogenate, either 5 µl of a solution of 4 mM cholesterol in ethanol (200 µM final concentration) or 4 µl of 270,000 dpm [4-¹⁴C]cholesterol in ethanol (final concentration 44 µM, specific activity 2.1 GBq/mmol) and either 100,000 dpm of UDP-[U-¹⁴C] glucose (final concentration 1.5 µM, specific activity 12.2 GBq/mmol) or 5 µl of 3.6 mM unlabelled UDP-glucose (final concentration 360 µM). After incubating for 30-120 min at 30°C, the reaction was terminated by the addition of 0.7 ml 0.45% NaCl solution and either 2 ml of ethyl acetate or 3 ml of chloroform/methanol 2:1. After vortexing and phase separation by centrifugation, the radioactivity in the organic phase was determined by scintillation counting or the extracted lipids were separated by TLC. The radioactivity on the silica gel plate was detected by radioscanning with a BAS-1000 Bio Imaging Analyser (raytest, Straubenhardt, Germany).

**Lipid Extraction and Analysis.** Expression cultures of *E. coli* and *P. pastoris* were grown and harvested as described above. The sedimented cells were boiled for 10 min in water and centrifuged again. Lipid extraction was performed with chloroform/methanol 1:2 (v/v) and chloroform/methanol 2:1 (v/v). The lipid extract was washed by Folch partitioning (Folch et al., 1957) (chloroform/methanol/0.45 % NaCl solution, 2:1:0.75) and the organic phase was evaporated. The residue was redissolved in chloroform/methanol 2:1 and subjected to analytical and preparative TLC. For NMR spectroscopy and mass spectrometry (MS), the purified glycolipids were acetylated (with acetic anhydride in pyridine, 1:1) overnight at room temperature and subjected to repurification by preparative TLC in diethyl ether.

### Legends

The invention is further demonstrated by the following figures and table:
**Figure 1** *H. pylori* follows a cholesterol gradient and extracts the lipid from epithelial membranes. a, Chemoattraction of *H. pylori* by cholesterol. Using a 3-tube system containing different cholesterol concentrations, Hp or medium alone, movement of Hp towards cholesterol for 4 h was determined by counting CFU/mL. CFU, colony-forming units. b, *H. pylori* absorbs cholesterol from epithelium. AGS cells were incubated with NBD-cholesterol (green, 3 h, 5 µg/mL), washed with MβCD (5 mg/mL) and infected with Hp for 180 min. Cells were fixed, stained with anti-*Hp* antibody (cy5, converted into white) and examined by confocal fluorescence microscopy. Overlay of *Hp* and cholesterol appears purple. Scale bars represent 10 µm (upper panel) and 2 µm (lower panel). **c, d,** *H. pylori* associates with cholesterol-rich membrane domains. **c**, AGS cells were cholesterol depleted with 5 mg/mL MβCD for 30 min or left untreated, washed and infected with *Hp*-GFP for 90 min. Cell lysates were fractionated by sucrose gradient centrifugation. Precipitates of each fraction were analyzed by Western blotting with an anti-Flotillin-1 antibody. **d**, Parallel to (c), GFP activity was detected in each fraction and is depicted as relative fluorescence units (RFU).
**Figure 2** *H. pylori* converts epithelial cholesterol into cholesteryl glucosides and destructs lipid rafts. **a**, AGS cells were infected with Hp for 2 h or 4 h, or left uninfected. Cell lysates were fractionated by sucrose gradient centrifugation and analyzed by enzymatic detection of cholesterol in each fraction. **b**, AGS cells were cholesterol-depleted with 5 mg/mL MβCD for 30 min or left untreated, washed and synchronized infected with Hp for 2 h or 4 h. Thin layer chromatography of isolated lipids were developed with chloroform/methanol/ammonia (40:10:4, v/v) and visualized with Bial's reagent. Ch: Cholesterol, αCAG: Cholesteryl-acyl-α-glucoside, αCG: Cholesteryl-α-glucoside. **c**, **d**, **e**, AGS cells were transfected with GPI-GFP (GPI-GFP) or control vector (EGFP), infected with *Hp* or *HpΔPAI* lacking pathogenicity island for 4 h. Membrane localization was verified with TIRF-microscopy (c). Cell lysates were fractionated by sucrose gradient centrifugation. Fluorometric detection of GFP in each fraction is depicted in relative fluorescence units (RFU) (e). Equal GPI-GFP expression was audited by Western blotting with an anti-GFP antibody (d).
**Figure 3** Cholesterol promotes phagocytosis, T cell activation and protection against *H*. *pylori* infection. **a**, Increase in cholesterol amplifies. phagocytosis. Hp were incubated with cholesterol for 30 min, washed and incubated with J774A.1 macrophages for 90 min. Total adherent bacteria were counted by plating macrophage lysates for CFU. Phagocytosed *Hp* were determined by killing extracellular bacteria with gentamicin and plating macrophage lysates for CFU. b, Cholesterol facilitates antigen-specific T cell activation. *Hp* were pre-incubated with cholesterol or left untreated, washed and used for synchronized infection of macrophages for 90 min. T cells from total *Hp* lysates immunized mice or naïve animals were cultivate with untreated, ConA, *Hp* protein or *Hp*-incubated macrophages. Activation of *Hp*-specific T cells was measured by [³H]thymidine uptake during proliferation and is shown as counts per minute (cpm). Blocking antibodies against MHC-I or MHC-II compared to isotype controls were used for inhibiting antigen recognition. **c**, **f,** Cholesterol mediates T cell dependent protection against *H. pylori* infection. C57BU6 or RAG1-/- mice received 2% or 0% cholesterol diet, started 2 weeks before infection. Infections were performed by oral gavage of 9.5 x 10⁸ bacteria. 6 mice were infected per time point. Colonization density of Hp76 were analyzed after 24 h, 2, 10 or 25 weeks post infection. CFU, colony-forming units. **d**, High cholesterol diet promotes immune response. Paraffin embedded sections at week 25 post infection were stained with Hematoxylin and Eosin. **e**, Transcriptome analysis of *H*. *pylori* infected gastric mucosal tissue shows differentially regulated genes in C57BU6 cholesterol diet group compared to control diet group.
**Figure 4** Cholesteryl glucosides protect *H. pylori* from phagocytosis. **a**, *Hp* were grown for 4 h, while subsequently incubated with cholesterol (62 µM, 31 µM) for 30 min, washed, grown for 0 h, 2 h or 4 h and incubated with J774A.1 macrophages for 90 min. Thin layer chromatography of isolated lipids was developed with chloroform/methanol/ammonia (40:10:4, v/v) and visualized with Bial's reagent. **b,** Total adherent bacteria were counted by plating macrophage lysates for CFU. Phagocytosed *Hp* were determined by killing extracellular bacteria with gentamicin and plating macrophage lysates for CFU. **c, d,** Cholesteryl-α-glucoside prevents phagocytosis. *Hp* were incubated with cholesterol, cholesteryl-α-glucoside or cholesteryl-α-glucoside (0.25 mM, solubilized in 2-hydroxypropyl-β-cyclodextrin) for 30 min, washed, and subsequently incubated with J774A.1 macrophages for 90 min. Isolated bacterial lipids were analyzed by TLC and visualized with Bial 's reagent (c). Total adherent bacteria were counted by plating macrophage lysates for CFU. Phagocytosed Hp were determined by killing extracellular bacteria with gentamicin and plating macrophage lysates for CFU (d).
**Figure 5** Cholesterol-α-glucosyltransferase is required for immune escape of *H. pylori.* **a,** HpΔ*421* mutants lack cholesteryl glucosides. After lipid extraction, the cholesteryl glucoside content of wild type, HpΔ*421* mutant or reconstitutant *H. pylori* (HpΔ*421*reconst.) was analyzed by TLC and visualized with α-naphthol. **b**, Cholesterol-α-glucosyltransferase triggers escape from phagocytosis. J774A.1 macrophages were infected with wild type, HpΔ*421* mutant or reconstitutant *H. pylori* for 90 min. Intra- and extracellular (green) and extracellular (white) Hp were determined by differential immunofluorescence staining. Overlay appears purple. Scale bars represent 10 µm. **c**, Cholesterol-α-glucosyltransferase of *H. pylori* prevents induction of T cell responses. Mouse macrophages were infected with wild-type, Δ421 mutant or reconstitutant *H. pylori* for 90 min. T cells from total *Hp* lysates immunized mice or naïve animals were cultivated with untreated, ConA, Hp protein or Hp-incubated macrophages. Activation of Hp-specific T cells was measured by [³H]thymidine uptake during proliferation and is shown as counts per minute (cpm). Blocking antibodies against MHC-I and MHC-II compared to isotype controls were used for inhibiting antigen recognition. **d,** Cholesterol α-glucosylation is vital for robust *H. pylori* infection *in vivo.* C57BU6 mice were orally infected with 9.5 x 10⁸ wild type, HpΔ421 mutant or reconstitutant *H. pylori.* Stomachs of experimental animals were collected to determine the bacterial burden by counting CFU 12 h, 24 h and 48 h after infection.
**Figure S1 a**, 3-tube system apparatus used to assess chemotaxis of *H*. *pylori.* **b**, As for Fig. 1 a, analyzing time dependency of chemotaxis at a constant cholesterol concentration of 250 µM, or medium alone. To exclude increase in CFU due to bacterial replication, 3,500 bacteria were incubated with 250 µM cholesterol (growth control). At time points indicated, control tubes were analyzed in addition to the tubes of the chemotaxis assay and revealed that altered CFU were not the consequence of bacterial replication in the presence of cholesterol. **c,** *H. pylori* does not follow a phosphatidylcholine or 17β-estradiol gradient. Chemotactic response of *Hp* using cholesterol, phosphatidylcholine or 17β-estradiol in a 3-tube system for 180 min. The contents of each tube were diluted and plated on agar plates for determining CFU/mL. CFU, colony-forming units.
**Figure S2** *H. pylori* takes up cholesterol from epithelial cells. AGS cells were incubated with NBD-cholesterol (green) (3 h, 5 µg/mL), washed with MβCD (5 mg/mL) and infected with Hp for 30, 90 or 180 min. Cells were fixed, stained with *anti-Hp* antibody (cy5, converted into white) and examined by confocal fluorescence microscopy. Overlay appears purple. Scale bars represent 10 µm (upper panel) and 2 µm (lower (zoom) panel).
**Figure S3** *H. pylori* fails to incorporate cholesterol from supernatant of NBD-cholesterol-loaded epithelial cells. Hp (P12) was incubated with supernatant from NBD-cholesterol-loaded AGS cells, NBD-cholesterol or left untreated and fixed for subsequent analyzing by flowcytometry. **a**, Dot blot analysis of Hp cells expressing GFP (gate 2, red). **b**, Dot blot analysis of *Hp, Hp* incubated with supernatant from NBD-cholesterol loaded AGS cells (3 h), and *Hp* treated with NBD-cholesterol (30 min, 1 µg/mL) as positive control. **c**, The histogram of the gated population in (b) depicts fluorescence intensity of untreated *Hp* (green), *Hp* incubated with supernatant from NBD-cholesterol loaded AGS cells (red), Hp treated with NBD-cholesterol (black) and *Hp*-GFP (blue). **d**, Quantification of green fluorescence intensity in (c) as FL-1 median.
**Figure S4** *H. pylori* colocalizes with GM1. AGS cells were infected with *Hp* for 90 min, fixed and stained with anti-*Hp* antibody (Cy3, red) and CT-B binding to glycosphingolipid GM1 (FITC, green). Samples were examined by confocal microscopy. Scale bars represent 10 µm (upper panel) and 2 µm (lower panel).
**Figure S5** Purification and structural identification of glucosylated cholesterol derivatives from *H. pylori (Hp).* Total lipids (TL) from *Hp* were subjected to TLC with chloroform/methanol/water (70:30:5, v/v), and visualized with α-naphthol/sulfuric acid. Purified glucolipids (αCAG, αCG, and αCPG) were acetylated before structural analysis by MS and NMR spectroscopy. All MS data are in full agreement with cholesteryl glucoside, cholesteryl-6-O-tetradecanoyl-glucoside and cholesteryl-phosphatidyl-glucoside (with 19:0 cyclopropyl and 14:0 fatty acyl chains in the phosphatidyl residue) (data not shown)⁷. The NMR data confirmed the α-configuration of the glucopyranose in all three lipids, which in two compounds carried the additional substituent at C6 (data not shown).
**Figure S6** Increase in endogenous *H. pylori* cholesterol intensifies phagocytosis. J774A.1 macrophages were infected with cholesterol-loaded (0.125 or 0.25 mM) Hp for 90 min, cells were fixed, and stained with anti-*Hp* antibody. Intra- and extracellular *Hp* (green) and extracellular *Hp* (cy5, converted into white) were determined by differential immunofluorescence staining. Overlay appears purple. The merged fluorescence picture depicted phagocytosed Hp in green (but not purple). Scale bars represent 10 µm (upper panel) and 2 µm (lower (zoom) panel).
**FIG. 6**. Comparison of amino acid sequences from enzymes of the glycosyltransferase family 4.
   A, Dendrogram showing similarities between GT4 polypeptides from plants and bacteria. Enzymes whose functions as diacylglycerol glycosyltransferase or monoglycosyldiacylglycerol glycosyltransferase have been confirmed experimentally are framed (Dörmann 1999, Berg 2001, Yu 2002, Edman 2003, Hölzl 2005 PCP). The glycosyltransferase from *H. pylori* studied in the present work is marked by an asterisk. The other sequences represent hypothetical polypeptides deduced from genomic sequences of the respective organisms. The dendrogram has been constructed from pairwise similarities of amino acid sequences using ClustalX (Thompson et al., 1997). The sequences used for the alignment are present at the Carbohydrate-Active Enzymes server at URL: http://afmb.cnrs-mrs.fr/CAZY/ and are from *Acholeplasma laidlawii* (Al-1, diacylglycerol glucosyltransferase, AAK38877; Al-2, diacylglycerol-3-glucose-(1→2)-glucosyltransferase, AAL83700), *Arabidopsis thaliana* (At-1, sulfolipid synthase, AAM18913, At-2 diacylglycerol-3-galactose-(1→6)-galactosyltransferase, AAD42378), *Borrelia burgdorferi* (NP_212588), *Clostridium thermocellum* (ZP_00311836); *Deinococcus radiodurans* (DR1225, NP_294949), *Helicobacter hepaticus* (NP _860207), *Lactobacillus johnsonii* (NP_965126), *Streptococcus pneumoniae* (Sp-1, diacylglycerol glucosyltransferase, NP_358576; diacylglycerol-3-glucose-(1→2)-galactosyltransferase ,NP_358575), *Thermotoga maritima* (TM0744, NP_228553).
   B. ClustalX alignment of the cholesterol-α-glucosyltransferase candidate HP0421 from *H. pylori* with bacterial diacylglycerol-α-glucosyltransferases, all of which are members of GT4 (Campbell 1997). Black regions indicate identical amino acids in all 5 sequences. Gray regions indicate identical amino acids in 3 or 4 sequences. The characteristic E*X*₇E motif present in the putative active site is marked by arrowheads. The C-terminal ends of the sequences were omitted.
**FIG. 7.** Expression of HP0421 in *P. pastoris* resulted in the biosynthesis of ergosteroi-β-glucoside. Lipid extracts from different *P. pastoris* strains were separated by TLC in chloroform/methanol 85:15.
   The wildtype strain (WT) contained ergosterol-β-glucoside (βEG) and glucosylceramide (GlcCer). A double knock-out mutant (DKO) deficient both in sterol glucosyltransferase and ceramide glucosyltransferase activities lacked these glycolipids (Hillig 2003). The DKO mutant expressing HP0421 from *H. pylori* (DKO *Hp)* synthesized the novel glycolipid ergosterol-α-glucoside (αEG). In contrast, the DKO mutant expressing the ORF TM0744 from *Thermotoga maritima* (DKO *Tm)* contained diacylglycerol-α-glucoside (αDG) (Hölzl 2005 PCP).
**FIG. 8.** Recombinant HP0421 from *H. pylori* expressed in *E*. coli synthesized cholesterol-α-glucoside *in vitro.* Cell-free homogenates from transgenic *E. coli* expressing either the sterol-α-glucosyltransferase from *H. pylori (Hp)* or the sterol-α-glucosyltransferase from the plant *Avena sativa (As)* (Warnecke 1997) were used for *in vitro* enzyme assays. The assay mixtures contained either UDP-glucose and radiolabelled cholesterol ([¹⁴C]Chol) or radiolabelled UDP-glucose ([¹⁴C]Glc) and cholesterol. The labelled lipophilic reaction products were separated by high performance thin-layer chromatography in chloroform/methanol 80:20 and detected by radioscanning. Cholesterol-α-glucoside and cholesterol-β-glucoside (αCG and βCG) were visualized by radioscanning and identified by co-chromatography with authentic standards. Extracts from *E.* coli harboring the control plasmid did not show sterol glucosyltransferase acivity (data not shown).
**Fig. 9.** In vitro enzyme assay of recombinant Helicobacter cholesteryl alpha glucosyltransferase HP0421 expressed in E. coli. The assay was performed as described in "In Vitro Sterol Glucosyltransferase Assay" in the methods section. Briefly, cholesterol and UDP-glucose were incubated for 30 min in the presence of cholesteryl alpha glucosyltransferase (E. coli cell homogenate). Readout is the amount of cholesterol-alpha-glucoside formed during the incubation. The assay was performed in the absence (control) or in the presence of 125 µM up to 1 mM of PNP-GlcNAc, UDP-GlcNAc and GlcNAc which do not inhibit the cholesteryl alpha glucosyltransferase. In further control experiments, the amount of cholesterol glucoside formed during the incubation period dose-dependently decreased in the presence UDP or glucose (125 µM up to 1 mM), as expected for a competitive inhibition.
   **Table I.** Acceptor specificity of the cholesterol-α-glucosyltransferase expressed in *E. coli.* Reaction mixtures contained cell-free homogenate from *E. coli* cells, UDP-[¹⁴C]glucose and unlabelled acceptor. Incorporation with UDP-glucose and cholesterol was taken as 100 %. Values represent means from two independent determinations.

## Claims

1. Use of an inhibitor of a cholesteryl alpha-glucosyltransferase for the manufacture of a medicament for the prevention or/and treatment of a Helicobacter infection or/and a Helicobacter associated disease.

2. The use of claim 1, wherein the cholesteryl alpha-glucosyltransferase is a Helicobacter cholesteryl alpha-glucosyltransferase, a homologue or/and a fragment thereof, preferably Helicobacter cholesteryl alpha-glucosyltransferase HP0421, jhp0963, HH0676, a homologue or/and a fragment thereof.

3. The use of claims 1 or 2, wherein the Helicobacter associated disease is selected from chronic gastritis, peptic ulcer disease, and cancer such as MALT lymphoma or gastric adenocarcinoma.

4. The use of any of the preceding claims, wherein the medicament is for combination with a further therapy for the prevention or/and treatment of a Helicobacter infection or/and Helicobacter associated disease.

5. The use of claim 4, wherein the further therapy comprises the administration of at least one antibiotic.

6. The use of claims 4 or 5, wherein the further therapy comprises a stimulation of the immune system, such as an unspecific or a Helicobacter specific stimulation of the immune system.

7. The use of claim 6, wherein the further therapy is a vaccination.

8. The use of any of the preceding claims, wherein the inhibitor is selected from antibodies, antibody fragments, anti-sense molecules, siRNA molecules, ribozymes, proteinaceous and low molecular weight compounds.

9. The use of any of the preceding claims, wherein the medicament is for combination with a cholesterol-rich diet.

10. A pharmaceutical composition comprising a cholesteryl alpha glucosyltransferase, a nucleic acid encoding a cholesteryl alpha glucosyltransferase, a fragment or/and a homologue thereof, optionally together with a pharmaceutically acceptable carrier, diluent or/and adjuvant.

11. The pharmaceutical composition of claim 10, which is an immunogenic composition.

12. The pharmaceutical composition of claims 10 or 11, which is a vaccine.

13. The pharmaceutical composition of any of the claims 10 to 12, wherein the cholesteryl alpha-glucosyltransferase is a Helicobacter cholesteryl alpha-glucosyltransferase, a fragment or/and a homologue thereof, preferably Helicobacter cholesteryl alpha-glucosyltransferase HP0421, jhp0963, HH0676, a fragment or/and a homologue thereof.

14. The pharmaceutical composition of any of the claims 10 to 13, wherein the nucleic acid encoding the cholesteryl alpha-glucosyltransferase comprises the sequence SEQ ID NO:1, 3 or/and 5, a sequence capable of hybridizing under stringent conditions with SEQ ID NO:1, 3 or/and 5, a sequence within the scope of the degeneracy of the genetic code of SEQ ID NO:1, 3 or/and 5, a fragment or/and a homologue thereof.

15. The pharmaceutical composition of any of the claims 10 to 14 for the prevention or/and treatment of a Helicobacter infection or/and a Helicobacter associated disease.

16. The pharmaceutical composition of any of the claims 10 to 15 for combination with a further therapy for the prevention or/and treatment of a Helicobacter infection or/and a Helicobacter-associated disease.

17. The pharmaceutical composition of any of the claims 10 to 16 for combination with a cholesterol-rich diet.

18. A screening method for the identification of an agent for the prevention or/and treatment of a Helicobacter infection or/and a Helicobacter associated disease, comprising identifying a cholesteryl alpha-glucosyltransferase inhibitor.

19. The screening method of claim 18 wherein identifying the cholesteryl alpha-glucosyltransferase inhibitor comprises the steps
(a) contacting at least two compounds with a cholesteryl alpha-glucosyltransferase or/and with a nucleic acid encoding a cholesteryl alpha-glucosyltransferase,
(b) determining the cholesteryl alpha-glucosyltransferase activity, and
(c) selecting a compound of (a) capable of inhibiting the cholesteryl alpha-glucosyltransferase activity in (b).

20. The screening method of claim 18 or 19, wherein the cholesteryl alpha-glucosyltransferase inhibitor is identified in a molecular or/and cellular assay.

21. The screening method of any of the claims 18 to 20, wherein the cholesteryl alpha-glucosyltransferase is a Helicobacter cholesteryl alpha-glucosyltransferase, preferably Helicobacter cholesteryl alpha-glucosyltransferase HP0421, jhp0963, HH0676, a fragment or/and a homologue thereof.

22. The screening method of any of the claims 18 to 21, wherein the nucleic acid encoding the cholesteryl alpha-glucosyltransferase comprises SEQ ID NO:1, 3 or/and 5, a sequence capable of hybridizing under stringent conditions with SEQ ID NO:1, 3 or/and 5, a sequence within the scope of the degeneracy of the genetic code of SEQ ID NO:1, 3 or/and 5, a fragment or/and a homologue thereof.

23. Use of a cholesteryl alpha-glucosyltransferase, a nucleic acid encoding a cholesteryl alpha-glucosyltransferase, a fragment or/and a homologue thereof for the identification of an agent for the prevention or/and treatment of a Helicobacter infection or/and a Helicobacter associated disease.

24. A method for prevention or/and treatment of a Helicobacter infection or/and a Helicobacter associated disease comprising administering an effective amount of a medicament as defined in any of the claims 1 to 9 or/and a pharmaceutical composition of any of the claims 10 to 17 to a subject in need thereof.

25. The method of claim 24 combined with a further therapy for the prevention or/and treatment of a Helicobacter infection or/and a Helicobacter-associated disease.

26. The method of claim 24 or 25 combined with a cholesterol-rich diet.

27. A method for enhancing an immune response against a Helicobacter infection comprising administering a cholesterol-rich diet to a subject in need thereof.

28. The method of claim 27, wherein enhancing the immune response is increasing phagocytosis of Helicobacter cells by antigen presenting cells or/and increasing T cell activation.

29. The method of claim 27 or 28, further comprising inhibiting cholesterol degradation or/and conversion into cholesterol glucosides in a *Helicobacter* cell.
